# EUROPEAN PATENT SPECIFICATION

(11) **EP 2 286 794 B1**
(45) Date of publication and mention of the grant of the patent: **30.03.2016**
(21) Application number: 10180750.1
(22) Date of filing: 15.10.2004
(51) Int. Cl.: A61K 9/127, A61K 31/335, A61P 35/00, A61K 31/00

(54) **USE OF CATIONIC LIPOSOMES COMPRISING PACLITAXEL**
VERWENDUNG VON KATIONISCHEN LIPOSOMEN MIT PACLITACEL
UTILISATION DE LIPOSOMES CATIONIQUES AVEC PACLITAXEL

(30) Priority: 15.10.2003 EP 03023539; 09.01.2004 EP 04000361
(43) Date of publication of application: 23.02.2011
(62) Divisional of application: 04790494.1
(73) Proprietor: MediGene AG, 82152 Planegg (DE)
(72) Inventor: Teifel, Michael, 64331, Weiterstadt (DE); Michaelis, Uwe, 82362, Weilheim (DE); Sauer, Birgitta, 82166, Gräfelfing (DE); Bartelheim, Kerstin, 82269, Walleshausen (DE); Brunner, Christoph, 82387 Antdorf (DE)
(74) Representative: Weickmann & Weickmann

(56) References cited:
- WO-A-01/17508
- WO-A-98/40052
- WO-A-2004/002455
- WO-A-2004/002468
- US-A- 6 090 955
- CAMPBELL R B ET AL: "INFLUENCE OF CATIONIC LIPIDS ON THE STABILITY AND MEMBRANE PROPERTIES OF PACLITAXEL-CONTAINING LIPOSOMES" JOURNAL OF PHARMACEUTICAL SCIENCES, AMERICAN PHARMACEUTICAL ASSOCIATION. WASHINGTON, US, vol. 90, no. 8, August 2001 (2001-08), pages 1091-1105, XP001101828 ISSN: 0022-3549
- EMERSON D L: "Liposomal delivery of camptothecins" PHARMACEUTICAL SCIENCE AND TECHNOLOGY TODAY, ELSEVIER TRENDS JOURNALS, CAMBRIDGE,, GB, vol. 3, no. 6, 1 June 2000 (2000-06-01), pages 205-209, XP002235822 ISSN: 1461-5347

## Description

The present invention relates to the use of pharmaceutical preparations comprising paclitaxel for administration to a human patient in need thereof.

The use of antimitotic drugs, such as taxanes, as therapeutic agents for human patients suffering from diseases which are connected with enhanced mitosis are well known in the art.

Paclitaxel has a unique mechanism of action and a broad spectrum of antiproliferative activity because paclitaxel binds to microtubules and promotes tubulin polymerisation and stabilizes the assembled microtubules. As a result, paclitaxel blocks the cell cycle at prophase resulting in an accumulation of cells in the G2/M phase.

Unfortunately, paclitaxel has extreme low solubility in water, which makes it difficult to provide a suitable dosage form. Currently, paclitaxel is formulated and administered in a vehicle containing Cremophor EL (a polyethoxylated castor oil) and ethanol in a 50:50 (vol/vol) ratio. This solution is diluted 1:10 in saline before being administered to humans. However, various severe side reactions, such as hypersensitivity and hypertensive reactions, nephrotoxicity and neurotoxicity, for example, have been reported in patients due to Cremophor EL formulation.

Further, even though paclitaxel (among other antitumor drugs) is a potent, well-established standard antitumor drug ({Rowinsky, 1995 #1}, {Awada, 2002 #2}, {Seidman, 2003 #3}, {Romanini, 2003 #4}), drug-unresponsive tumors and metastases are observed frequently in cancer patients ({Blom, 1996 #5}, {Modi, 2002 #6}, {Ozols, 2003 #7}). Genetically instable, rapidly dividing tumor cells gain the capacity to overcome the growth inhibitory effect of a selected anti-cancer drug ({Vogelstein, 1988 #8}, {Kerbel, 1991 #9}). This capacity is usually not limited to a single drug (first line) but extends to other drugs which are used after development of the first resistance. Hence, this phenomenon is called multi drug resistance (MDR). As the number of available and approved anti-neoplastic drugs is very limited for many cancer types, many patients succumb since their cancer tissues express MDR. The obvious problem, therefore, is to find methods and means to kill drug-resistant tumors, especially drug resistant cells, which are already resistant against the respective drug.

A number of approaches were taken to deal with the above mentioned problems. The conventional strategy is to increase doses up to the maximal tolerated dose (MTD) and attempt to eradicate all tumor cells as quickly and completely as possible ({Schünemann, 1999 #10}, {Heidemann, 1997 #11}). It is obvious that this strategy causes severe side effects and can not be extended to longer periods. Therefore, this treatment schedule consists of cycles of one short treatment period (usually 1 day - 1 week) at MTD and a treatment-free interval of several weeks (usually 3-4 weeks), to allow the patient to recover from the obligatory side effects ({Schünemann, 1999 #10}, {Heidemann, 1997 #11}, {Romanini, 2003 #4}). In many instances, tumor growth can also restart during these drug-free periods. Most importantly, this approach fails in many patients where tumor cells develop a high level of resistance which enables them to accommodate with drug concentrations at the MTD. The patients become therapy refractory.

The most common solution is to start treatment with a second drug ({Blom, 1996 #5}, {Awada, 2002 #2}, {Seidman, 2003 #3}, {Heinemann, 2003 #12}, {Thigpen, 2003 #13}). In the best case, the second line treatment is successful and the patient is cured. A common experience however is that tumors only respond for a certain time leading to a temporary regression of the tumor. After that, tumors become also resistant to the second drug. Continuing with this strategy leads to development of multi drug resistant tumors which are finally refractory to all available anti-cancer drugs ({Blom, 1996 #5}, {Seidman, 2003 #3}, {Thigpen, 2003 #13}).

Another possibility is to treat patients immediately with a combination of 2 or more drugs ({Heinemann, 2003 #12}, {Kuenen, 2002 #14}, {Sledge, 2003 #15}, {Ozols, 2003 #7}, {Reck, 2003 #17}, {Romanini, 2003 #4}). This strategy can be more successful as it decreases the likelihood for development of a double drug resistance. However, this strategy needs to explore time and cost intensively suitable drug combinations. A second disadvantage is that the side effects may also increase ({Kuenen, 2002 #14}, {Ozols, 2003 #7}). The therapeutic window concomitantly becomes small and the toxic effects may overlay the envisioned therapeutic benefit. Also in this case, multi drug resistance may develop and the therapy becomes ineffective ({Zimpfer-Rechner, 2003 #18}, {Sledge, 2003 #15}, {Sledge, 2003 #16}, {Ozols, 2003 #7}).

The consequence of the negative experiences with such traditional treatment strategies is to develop more and more new drugs to extend the above described treatment options. Obviously, it is a very time and cost intensive race for more potent drugs which will eventually lead in many cases to therapy refractory tumors. In recent years, this recognition has led to a new approach to circumvent tumor resistance. It is based on the assumption that the MDR is caused by overexpression of enzymes which enable cells to expel chemotherapeutic drugs. The most famous member of this category of enzymes is called p-glycoprotein (p-gp). It is located in the cytoplasmic membrane and exports in an ATP-driven way ({Nobmann, 2001 #19}, {Thomas, 2003 #20}) compounds like paclitaxel or doxorubicin ({Harker, 1985 #21}, {Fellner, 2002 #22}, {Kiesewetter, 2003 #23}). This notion led to the development of p-gp inhibitors which are meant to reverse p-gp mediated drug resistance. Hence the term chemosensitizers was coined for this class of molecules. One of the first examples tested was verapamil. Clinical studies, however, revealed unsatisfactory results, possibly due to to low specific activity ({Thomas, 2003 #20}, {Kohler, 2003 #24}). The further research led to a second generation of compounds which again were found not to be clinically applicable ({Leonard, 2002 #25}, {Thomas, 2003 #20}). Today a few substances of the third generation, one known as tariquidar, are in clinical trials ({Agrawal, 2003 #26}, {Callies, 2003 #27}). The usefulness and broad applicability of these compounds is, however, still unclear ({Leonard, 2002 #25}, {Thomas, 2003 #20}). Even though much improved in comparison to first generation chemosensitizers, third generation compounds also cause side effects and may have unforeseen consequences for the whole body. Extensive clinical testing is needed and it is so far uncertain if such approaches can become general practice in the future ({Leonard, 2002 #25}, {Thomas, 2003 #20}).

Different delivery systems have been used to enhance the effect of paclitaxel and/or reduce toxicity. Liposomes are one of many carriers that have been developed to enhance aqueous solubility and thus efficiency, combined with less toxicity.

U.S. Pat. No. 5,648,090, U.S. Pat. No. 5,424,073 and U.S. Pat. No. 6,146,659 (Rahman et al.) provide a liposomal encapsulated paclitaxel for a method for treating cancer in mammals. These patents disclose a method of administering to the host a pharmaceutical composition of a therapeutically effective amount of liposomes which include a liposome forming material, cardiolipin, and an agent such as paclitaxel, or an antineoplastic derivative of paclitaxel, or a mixture thereof, with a pharmaceutically acceptable excipient. In U.S. Pat. No. 6,146,659, a method of administering a taxane to a patient is provided by administering taxane over a period of less than an hour in an amount from about 75 to 300 mg/m², wherein the taxane is liposomally encapsulated. The liposomes disclosed therein are negatively charged.

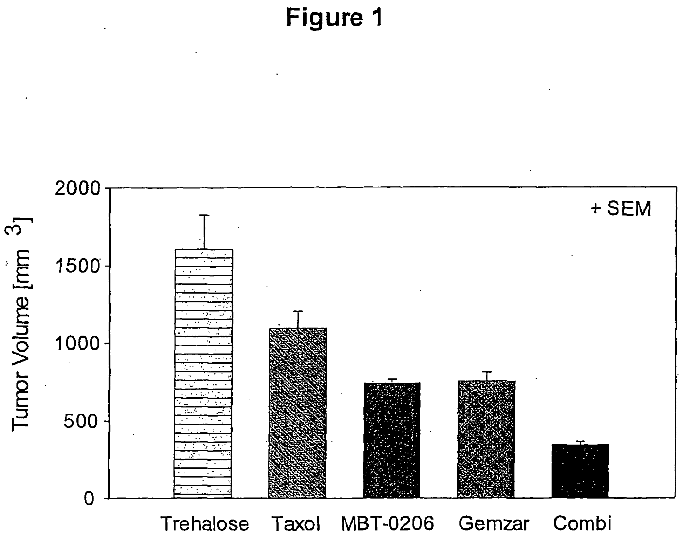

U.S. Pat. No. 6,090,955 relates to liposome-encapsulated taxol also. The liposomes disclosed in the examples consist entirely of the neutral lipid egg phosphatidyl choline. The liposomes are used for animal experiments. On a human breast cancer (MaTu) it is shown that the liposome preparation, already after a single administration of 50 mg/kg, exhibits a better therapeutic effectiveness and a lower hemotoxicity than free taxol (4 days treatment, 12.5 kg/mg per day) (Table 1 and FIG. 1).

Since the disclosure of McDonald et al., U.S. Pat. No. 5,837,283, WO 98/40052 and WO 01/17508 it is known that positively charged liposomes specifically target angiogenic endothelial cells. In this connection, Campbell et al., 2001, J. Pharm. Sci., Am Pharm. Ass. Washington, pp. 1091-1105, evaluated the influence of cationic lipids on the stability and membrane properties of paclitaxel-containing liposomes. Clinical data on human patients are not presented.

WO 2004/002455 relates to a method of stabilizing a low molecular weight compound in a cationic liposome, wherein said compound has a low solubility in a lipid membrane and/or a low permeability across a lipid membrane. Lipophilic active agents such as paclitaxel are not disclosed. A defined monthly dosage is not disclosed.

WO 2004/002468) relates to a method of producing a cationic liposomal preparation comprising a lipophilic compound. A dose regimen for a human patient is not disclosed.

Emerson, 2000, Pharm. Sci. and Techn. Today, Elsevier Trends J. Cambridge, pp. 205-209, discloses the liposomal delivery of camptothecins. The liposomes are comprised of cholesterol, a phosphatidylcholine, and an excipient.

Thus, the problem underlying the present invention was to provide a method of administering paclitaxel to a subject in need thereof in a therapeutically effective amount without severe side effects. Particularly, the occurrence of side effects caused by the necessity of using high initial treatment doses of paclitaxel in the application of prior art preparations such as Cremophor preparations should be avoided.

In the present application, clinical data on human patients of the administration of cationic liposomal preparations containing paclitaxel are presented.

Surprisingly, it was found that administration of a cationic liposomal preparation having a high content of paclitaxel as an active ingredient selectively affects angiogenic endothelial cells in a human patient in need thereof. Thus, in a first aspect, the invention comprises administering to a patient a cationic liposomal preparation comprising from about 30 mole% to about 98 mole% cationic lipid, paclitaxel in an amount of at least about 0.1 mole% mole% and neutral and/or anionic lipids from about 0 mole % to about 70 mole% in the treatment of an angiogenesis-associated condition selected from wound healing, cancer, an inflammatory disease, a chronic inflammatory disease selected from rheumatoid arthritis, dermatitis, psoriasis or endometriosis in an amount of about 0.01 to 2,5 mg of paclitaxel / kg body weight of said patient per single unit dose at a monthly dose of about 0.25 mg up to about 60 mg of paclitaxel / kg body weight of said patient.

The term "about" as used in the present specification describes a deviation from the given value of plus or minus 5%.

The above formulations are particularly suitable for the prevention and/or treatment of multi drug resistant tumors and/or tumor metastases, optionally in combination with other treatment protocols.

The advantages of the present invention are as follows:
- high amounts of active ingredient
- selective targeting
- improved efficacy
- avoiding multi drug resistance (different target)
- affecting drug resistance by killing resistant cells directly
- affecting metastasis
- lower side effects compared to traditional chemotherapy or with neutral or anionic liposomes
- reduction of disease related pain
- improvement of quality of life
- stabilization of body weight during treatment
- synergistic effects with traditional therapy regimes

The present pharmaceutical composition can be administered at a monthly dose of 0.25 mg up to 60 mg of liposomal paclitaxel / kg body weight (bw) of a patient, preferably of about 0.5 mg up to about 30 mg of liposomal paclitaxel / kg bw and more preferably of about 1.0 mg up to about 15 mg of liposomal paclitaxel / kg bw. On an average, a human patient has about 70 kg body weight and is about 172 cm tall.

The dose scheme can range from a plurality of times daily to a plurality of times during a month period, each of said times being separated by an interval of between one day and 3 weeks. The total treatment period is preferably at least one month.

The pharmaceutical composition is also suitable for a long-term administration for at least 3 months, for at least 4 months, for at least 6 months or for at least 12 months and up to 6 months, up to 12 months, up to 18 months, up to 24 months or even longer.

Even in long-term treatments, the drug resistances or detrimental side-effects like alopecia, nephropathy or others usually do not occur. Further, usually no premedication like corticosteroids or anti-histamines is required.

The present pharmaceutical composition can be administered at a single unit dose scheme of about 0.01 to about 2.5 mg, preferably about 0.05 to about 2.5 mg liposomal paclitaxel per kg of body weight. Preferably, 0.1 to 2.5 mg liposomal paclitaxel per kg of body weight per single unit dose is administered.

In a further preferred embodiment, about 1 to about 10 mg liposomal paclitaxel/kg bw per monthly dose is administered. In a still further preferred embodiment about 20 to about 60 mg liposomal paclitaxel kg bw per monthly dose is administered. In a still further preferred embodiment about 1 to about 7.5 mg liposomal paclitaxel/kg bw per monthly dose is administered.

The suitable dose of liposomal paclitaxel for application to a human patient is in an amount of about 0.01 to 2.5, preferably 0.02 to 1.7, and more preferably 0.05 to 0.5 mg/kg bw at least once a day, e.g. twice, three times or more each day; about 0.01 to 5.0, preferably 0.02 to 2.5 and more preferably 0.05 to 1.7 mg/kg bw every other day; about 0.01 to 2.5, preferably 0.02 to 2.5 and more preferably 0.05 to 2.5 mg / kg bw once a week.

The monthly dose is preferably administered in a plurality of single dose units. The administration of a plurality of low doses may be at least as effective as the administration of a single high dose. During the treatment interval the dose units and the dose intervals may remain constant. On the other hand, the dose units may be increased during the treatment interval, e.g. beginning with a starting dose and escalating in one or several steps to a consolidation dose, which may be 3 or 4 or more times higher than the starting dose. Additionally or alternatively, the treatment interval between single doses may be altered, e.g. decreased or increased during the treatment period.

The preferred dose for a single administration is about 0.25 to about 1.75 mg/kg bw.

A further preferred treatment protocol comprises administering said cationic liposomal preparation is
(i) at least 3 times, especially 3-5 times in a first week, followed by an interval of 1-3 weeks without administration, and optionally one or several repeats of this protocol, wherein the monthly dose is preferably about 1 to about 10 mg/kg bw, especially about 5 to about 7.5 mg/kg bw,
(ii) once in a first week followed by an interval of at least one week, especially 1-3 weeks, without administration, and optionally one or several repeats of this protocol, wherein the monthly dose is preferably about 20 to about 60 mg/kg bw.
(iii) once in a week for one week or several successive weeks, preferably for at least 4 weeks, wherein the monthly dose is preferably about 1 to about 7.5 mg/kg bw, especially about 3 to about 6 mg/kg bw, or
(iv) a combination of (i), (ii) and/or (iii).

For applications in human medicine, the present pharmaceutical composition may be administered at a monthly dose of preferably about 9 mg up to about 3700 mg, especially up to about 2237 mg/m² human body surface (bs), of about 18 up to about 1168 mg/m² bs and of about 37 mg up to about 584 mg/m² bs. On an average a human patient has a body surface of about 1.84 m². Thus, for an average person of 70 kg body weight and 172 cm height, preferred values for monthly doses, single doses etc. which have been indicated above in mg/kg body weight (bw) may be converted for human applications to corresponding values of in mg/m² human body surface (bs) by multiplication with a species-specific factor according to known methods.

In an even further aspect, the cationic liposomal preparation of the present invention comprises at least one cationic lipid from about 30 mole% to about 99.9 mole%, preferably to about 98 mole% cationic lipid, paclitaxel in an amount of at least about 0.1 mole%, preferably of at least about 2 mole%; and at least one neutral and/or anionic lipid from about 0 mole % to about 70 mole% for manufacturing a pharmaceutical composition for simultaneous, separate, or sequential combination therapy with a jointly effective dose of at least one further active agent, which is selected from antineoplastic agents, especially antimitotic agents selected from paclitaxel, alkylating agents, especially platinum containing compounds selected from cisplatin, carboplatin, DNA topoisomerase inhibiting agents selected from camptothecin or doxorubicin, RNA / DNA antimetabolites, especially 5-fluorouracil or gemcitabine and other compounds having antitumour activity, or a compound that reduces or eliminates hypersensitivity reactions comprising steroids, antihistamines, H2 receptor antagonists, and combinations thereof in a sufficient amount to prevent fatal anaphylactic reactions, or a compound comprising Ranitidine, Dexamethasone, Diphenhydramine, Famotidine, Hydrocortisone, Clemastine, Cimetidine, Prednisolone, Chlorpheniramine, Chlorphenamine, Dimethindene maleate, and Promethazine, or a chemosensitizer comprising cell cycle modulators, substances that revert a drug resistance selected from verapamil, vasoactive substances selected from anti-hypertensive drugs, substances that modify interactions of cationic liposomes with blood components selected from protamine, and/or heat and/or radiation and/or cryotherapy, wherein the single unit dose of liposomal paclitaxel is in an amount of about 0.01 to 2.5 mg of paclitaxel / kg body weight of said patient.

In a preferred embodiment, the liposomal preparation comprises paclitaxel in an amount of about 0.1 mole%, particularly of about 2 mole%, to about 8 mole%, preferably in an amount of about 0.5 mole%, particularly of about 2 mole%, to about 5 mole%, more preferably in an amount of about 1 mole% to about 4 mole% and most preferably in an amount of about 2.5 mole% to about 3.5 mole%. The cationic liposomal preparation of the present invention comprises substantially no paclitaxel crystals.

The liposomal preparation of the present invention is a cationic liposomal preparation which comprises cationic lipids in an amount of about 30 mole% to about 99.9 mole%, particularly to about 70 mole%, preferably from about 40 mole% to about 60 mole% and most preferably from about 45 mole%, to about 55 mole%. The preparation and the cationic lipids are characterized by having a positive zeta potential in about 0.05 M KCl solution at about pH 7.5 at room temperature.

The preferred cationic lipids of the liposomal preparation are N-[1-(2,3-dioleoyloxy)propyl]-N,N,N-trimethyl ammonium salts, e.g. the methylsulfate (DOTAP). Other useful lipids for the present invention may include:
DDAB, dimethyldioctadecyl ammonium bromide; 1,2-diacyloxy-3-trimethylammonium propanes, (including but not limited to: dioleoyl, dimyristoyl, dilauroyl, dipalmitoyl and distearoyl; also two different acyl chain can be linked to the glycerol backbone); N-[1-(2,3-dioloyloxy)propyl]-N,N-dimethyl amine (DODAP); 1,2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N,N-trimethylammonium chloride (DOTMA); 1,2-dialkyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS); 3β-[N-(N',N'-dimethylamino-ethane)carbamoyl]cholesterol (DC-Chol); 2,3-dioleoyloxy-N-(2-(sperminecarboxamido)-ethyl)-N,N-dimethyl-1-propanaminium trifluoroacetate (DOSPA); β-alanyl cholesterol; cetyl trimethyl ammonium bromide (CTAB); diC14-amidine; N-*tert*-butyl-N'-tetradecyl-3-tetradecylamino-propionamidine; 14Dea2; N-(alpha-trimethylammonioacetyl)didodecyl-D-glutamate chloride (TMAG); O,O'-ditetradecanoyl-N-(trimethylammonio-acetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl(alkenyl)-3-(2-hydroxyethyl)-imidazolinium chloride derivatives as described by Solodin et al. (1995) Biochem. 43:13537-13544, such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, containing a hydroxyalkyl moiety on the quaternary amine, as described e.g. by Felgner et al. [Felgner et al. J. Biol. Chem. 1994, 269, 2550-2561] such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. [US5498633]; cationic triesters of phospahtidylcholine, i.e. 1,2-diacyl-sn-glycerol-3-ethylphosphocholines, where the hydrocarbon chains can be saturated or unsaturated and branched or non-branched with a chain length from C₁₂ to C₂₄, the two acyl chains being not necessarily identical.

In a preferred embodiment, the liposomal preparation optionally comprises at least one neutral and/or anionic lipid. Neutral lipids are lipids which have a neutral net charge. Anionic lipids or amphiphiles are molecules which have a negative net charge. These can be selected from sterols or lipids such as cholesterol, phospholipids, lysolipids, lysophospholipids, sphingolipids or pegylated lipids with a neutral or negative net change. Useful neutral and anionic lipids thereby include: phosphatidylserine, phosphatidylglycerol, phosphatidylinositol (not limited to a specific sugar), fatty acids, sterols, containing a carboxylic acid group for example, cholesterol, 1,2-diacyl-sn-glycero-3-phosphoethanolamine, including, but not limited to, DOPE, 1,2-diacyl-glycero-3-phosphocholines and sphingomyelin. The fatty acids linked to the glycerol backbone are not limited to a specific length or number of double bonds. Phospholipids may also have two different fatty acids. Preferably the further lipids are in the liquid crystalline state at room temperature and they are miscible (i.e. a uniform phase can be formed and no phase separation or domain formation occurs) with the used cationic lipid, in the ratio as they are applied. In a preferred embodiment the neutral lipid is DOPC.

In a further preferred embodiment, the liposomal preparation comprises optionally neutral and/or anionic lipids, preferably DOPC in an amount of about 30 mole% to about 70 mole%, preferably from about 40 mole% to about 60 mole% and more preferably from about 45 mole% to about 55 mole %.

The cationic liposome preparation which is used herein can be dehydrated, stored for extended periods of time while dehydrated, and then rehydrated when and where it is to be used, without losing a substantial portion of its contents during the dehydration, storage and rehydration processes. To achieve the latter, one or more protective agents, such as cryoprotectants, may be present. Thus, the inventive cationic liposome preparation preferably comprises a cryoprotectant, wherein the cryoprotectant is selected from a sugar or an alcohol or a combination thereof. Preferably, the cryoprotectant is selected from trehalose, maltose, sucrose, glucose, lactose, dextran, mannitol or sorbitol.

In a further preferred embodiment, the liposomal preparation comprises trehalose in the range of about 5 % (m/v) to about 15 % (m/v) with respect to the total volume of the preparation.

The formulation of the cationic liposomes of the present invention may vary. In a preferred embodiment the molar ratio is 50:47:3 mole% of DOTAP, DOPC and paclitaxel. This formulation is also designated MBT-0206 or EndoTAG-1.

Liposomes of various sizes are useful in the present invention. In a preferred embodiment of the present invention cationic liposomes have an average particle diameter from about 25 nm to about 500 nm, preferably from about 50 to about 500 nm, more preferably from about 100 nm to about 300 nm.

The present liposome compositions can be administered systemically, preferably intravenously.

The cationic liposomes of the present invention may be used to treat any form of a condition associated with increased angiogenesis, such as cancer. The pharmaceutical composition of the present invention is particularly advantageous in treating tumors in human patients such as bladder cancer, breast cancer, colorectal cancer, endometrial cancer, leukaemia, lung cancer, lymphoma, melanoma, non-small-cell lung cancer, ovarian cancer, prostate cancer and to childhood cancers such as brain stem glioma, cerebellar astrocytoma, cerebral astrocytoma, ependymoma, Ewing's sarcoma/family of tumors, germ cell tumor, extracranial, Hodgkin's disease, leukaemia, acute lymphoblastic, leukaemia, acute myeloid, liver cancer, medulloblastoma, neuroblastoma, non-Hodgkin's lymphoma, osteosarcoma/malignant fibrous histiocytoma of bone, retinoblastoma, rhabdomyosarcoma, soft tissue sarcoma, supratentorial primitive neuroectodermal and pineal tumors, unusual childhood cancers, visual pathway and hypothalamic glioma, Wilms' Tumor and other childhood kidney tumors and to less common cancers including acute lymphocytic leukaemia, adult acute myeloid leukaemia, adult non-Hodgkin's lymphoma, brain tumor, cervical cancer, childhood cancers, childhood sarcoma, chronic lymphocytic leukaemia, chronic myeloid leukaemia, esophageal cancer, hairy cell leukaemia, kidney cancer, liver cancer, multiple myeloma, neuroblastoma, oral cancer, pancreatic cancer, primary central nervous system lymphoma, skin cancer, small-cell lung cancer, head & neck cancer, gall bladder and bile duct cancer, stomach cancer, gastrointestinal cancer, Kaposi's sarcoma, urothelial cell carcinoma, thyroid gland carcinoma, testicular carcinoma, vaginal cancer, angiosarcoma, soft tissue sarcoma and mesothelioma. Particularly, the cancer may be a mestastasing cancer and/or a standard (chemo)therapy-resistant cancer. Administration of the composition of the invention may slow or stop disease progression, or may lead to a partial or complete remission. Further conditions may be wound healing or an inflammatory disease or a chronic inflammatory disease such as rheumatoid arthritis, dermatitis, endometriosis or psoriasis.

The cationic liposomal preparations of the invention are particularly suitable for the treatment of cancer as indicated above, especially pancreatic cancer, inoperable pancreatic cancer, gastro-intestinal cancer, lung cancer, colorectal or gastric cancer, breast cancer, prostate cancer and melanoma, either as a monotherapy or a combination therapy with further treatment therapies, e.g. further active agents as indicated below in detail, especially with chemotherapeutic agents, e.g. DNA/RNA antimetabolites such as gemcitabine.

Surprisingly, it was found that active agents loaded into cationic liposomes act directly against endothelial or non-endothelial drug resistant cells, particularly drug resistant endothelial or non-endothelial tumor cells. Thus, the recent findings define non-endothelial drug resistant cells as a further target and thus enhance the anti-tumor applications of cationic liposomes. This is an additional aspect of the present invention.

Thus it is a further aspect of the present invention to use a cationic liposomal preparation comprising an active agent for the manufacture of a medicament against endothelial or non-endothelial drug resistant cells. The present invention also provides a method of administering a cationic liposomal preparation comprising an active agent to drug resistant cells of a subject in need thereof in a therapeutically effective amount to affect a disease such as cancer.

A further embodiment of this aspect relates to the use of a cationic liposomal preparation comprising at least one cationic lipid from about 30 mole% to about 99.9 mole%, an active agent in an amount of at least about 0.1 mole% and at least one neutral and/or anionic lipid from about 0 mole % to about 70 mole% for manufacturing a pharmaceutical composition for the prevention or treatment of disorders associated with and/or accompanied by the occurrence of drug resistant cells, e.g. for the prevention or treatment of drug-resistant tumors meaning especially the use as a second or third line treatment for cancer, especially pancreatic cancer, inoperable pancreatic cancer, gastro-intestinal cancer, lung cancer, colorectal or gastric cancer, breast cancer, prostate cancer and melanoma.

A major cause of primary treatment failure is the emergence of drug-resistant cell clones. Second-line treatment is administered after the initial administration has failed. A second-line chemotherapy may be a monotherapy using a different chemotherapeutic with a different mode of action or a combination of several further drugs or treatments. However, second line treatment might fail as well due to the development of multi-drug resistant cell clones. Thus, there might be third line treatment for such tumor recurrence. The cationic liposomal preparations are particularly suitable as a second or third line treatment as indicated above.

The cationic liposomal preparation of this aspect of the present invention comprises at least one cationic lipid from about 30 mole% to about 99.9 mole%, particularly to about 98 mole%, an active agent in an amount of at least about 0.1 mole%, particularly of at least about 2 mole%, and at least one neutral and/or anionic lipid from about 0 mole % to about 70 mole% for manufacturing a pharmaceutical composition for affecting drug resistant cells such that a disease associated with or accompanied by the occurrence of drug resistant cells is relieved (causing regression) or eventually cured.

It is a further surprising finding within the present invention that cationic liposomes comprising an active agent act alone or in combination with at least one other treatment therapy against metastasis formation.

Thus, it is a further object of the present invention to use a cationic liposomal preparation comprising an active agent for preparing a medicament against metastasis. The present invention also provides a method of administering a cationic liposomal preparation comprising an active agent to a subject in need thereof in a therapeutically effective amount to affect onset and/or progression of metastasis formation such as delaying and/or avoiding a metastatic disease. The term "affecting" as used herein generally means that a desired pharmacologic and/or physiologic effect is obtained, such as delaying and/or avoiding the onset and/or progression of a disease. In a preferred embodiment, the present invention is used for delaying and/or avoiding liver metastasis formation.

The cationic liposomal preparation of the present invention comprises at least one cationic lipid from about 30 mole% to about 99.9 mole%, particularly to about 98 mole%, a first active agent, e.g. paclitaxel in an amount of at least about 0.1 mole%, particularly of at least about 2 mole%, and at least one neutral and/or anionic lipid from about 0 mole % to about 70 mole% and is useful for manufacturing a pharmaceutical composition for simultaneous, separate, or sequential combination therapy with a jointly effective dose of at least one further active agent, e.g. a second non-liposomal active agent and/or heat and/or radiation and/or cryotherapy for delaying and/or avoiding metastasis formation.

The active agent loaded into the cationic liposomal preparation, e.g. an active agent for a monotherapy or the first active agent for a combination therapy, can be selected from a cytotoxic or cytostatic substance such as an anti-tumor or an anti-endothelial cell active substance, a chemotherapeutic agent or an immunological active substance. The active agent may also be selected from a taxane, a camptothecin, a statin, a depsipeptide, thalidomide, other agents interacting with microtubuli such as discodermolide, laulimalide, isolaulimalide, eleutherobin, Sarcodictyin A and B, and in a most preferred embodiment, it is selected from paclitaxel, docetaxel, camptothecin or any derivative thereof.

Thus, in a preferred embodiment of the present invention said liposomal preparation comprises a taxane, preferably paclitaxel or docetaxel or a derivative thereof in an amount of about 0.1 to about 20 mol%, preferably in an amount of about 0.5 mole% to about 10 mole%, more preferably in an amount of about 1 mole% to about 5 mole% and most preferably in an amount of about 2 mole% to about 4 mole%.

The at least one further active agent may be a cytotoxic or cytostatic substance as described above, such as an anti-tumor or an anti-endothelial cell active substance, a chemotherapeutic agent, an immunological active substance, a compound that reduces or eliminates hypersensitivity reactions or a chemosensitizer. Preferably, the at least one further active agent is present in a non-liposomal formulation. Further, it is preferred that the active agent and the further active agents are different.

The further active agents are selected from antineoplastic agents, especially antimitotic agents like paclitaxel, alkylating agents, especially platinum containing compounds like cisplatin, carboplatin, DNA topoisomerase inhibiting agents like camptothecin or doxorubicin, RNA/DNA antimetabolites, especially 5-fluorouracil or gemcitabine and/or other compounds having antitumor activity. Especially preferred are combination therapies with cisplatin or carboplatin or with 5-fluorouracil or with gemcitabine.

The compound that reduces or eliminates hypersensitivity reactions is selected from the group comprising (but not limited to) steroids, antihistamines, H2 receptor antagonists, and combinations thereof in a sufficient amount to prevent fatal anaphylactic reactions. The compound is selected from the group comprising Ranitidine, Dexamethasone, Diphenhydramine, Famotidine, Hydrocortisone, Clemastine, Cimetidine, Prednisolone, Prednison, Chlorpheniramine, Chlorphenamine, Dimethindene maleate, Indomethazine and Promethazine or any derivative thereof.

The chemosensitizer is selected from the group comprising (but not limited to) cell cycle modulators, substances that revert a drug resistance like verapamil, vasoactive substances like anti-hypertensive drugs, substances that modify the charge-related interaction of cationic liposomes with blood components like protamine.

It should be noted that the present disclosure of the invention also relate to all other aspects. This particularly refers to the amount and type of cationic lipid, the amount and type of neutral and/or anionic lipid, the amount and type of active agent, the amount and type of further active agent for combination therapy, and the type of disorder to be treated.

### Figure Legends

**Figure 1****:** Tumor Volume after End of Treatment
   Tumor size of L3.6pl pancreatic tumors 19 days after start of treatment. Treatment with 10% trehalose, paclitaxel, MBT-0206, Gemzar (gemcitabine) and the combination of both MBT-0206 and Gemzar started 8 days after tumor cell inoculation. Gemzar was applied i.p. at a dose of 100 mg/kg bw twice a week (Mon, Thu). paclitaxel and MBT-0206 were applied i.v. on a Mon, Wed, Fri schedule at a paclitaxel dose of 5 mg/kg bw. The combination group received both MBT-0206 and Gemzar with the respective schedule.
   Tumors were measured by palpation with a calliper on day 23 and 27. Mean ± SEM; n = 9 per group.

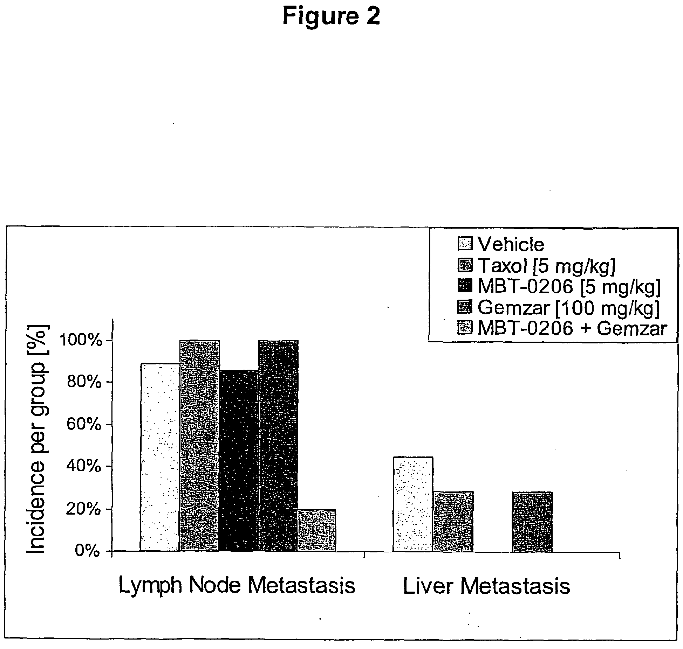

**Figure 2****:** Metastases after Therapy
   Metastases at day 19 after start of treatment. Treatment with 10% trehalose, paclitaxel, MBT-0206, Gemzar (gemcitabine) and the combination of both MBT-0206 and Gemzar started at day 8 after tumor cell inoculation. Gemzar was applied i.p. at a dose of 100 mg/kg bw twice a week (Mon, Thu). paclitaxel and MBT-0206 were applied i.v. on a Mon, Wed, Fri schedule at a paclitaxel dose of 5 mg/kg bw. The combination group received both MBT-0206 and Gemzar with the respective schedule, n = 9 per group..

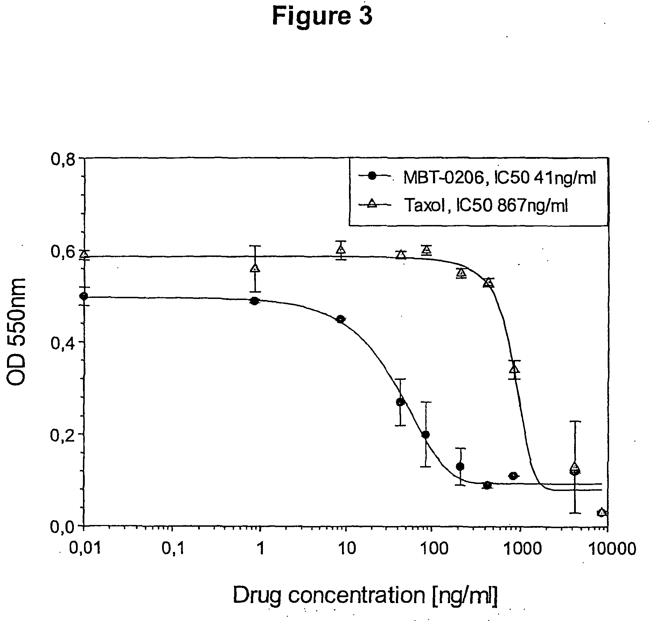

**Figure 3 - 5****:** The growth inhibitory assay was performed in 24-well plates with each drug concentration tested in duplicate (n=2 wells). 4 x 10⁴ cells per well were seeded into a 24-well plate and incubated over night. The following day, 10-11 concentrations of the respective drug formulation were added for 72 h to cover the range depicted in the respective graphs. Finally, the cell viability was determined by a standard MTT-assay measuring the activity of mitochondrial dehydrogenases.
   **Figure 3****:** Inhibitory Potential of MBT-0206 and paclitaxel against the highly drug-resistant uterus sarcoma line Mes-SA/Dx-5_{MBT}.
   **Figure 4****:** Inhibitory Potential of MBT-0206 and paclitaxel against the moderatly drug-resistant uterus sarcoma line Mes-SA/Dx-5.
   **Figure 5****:** Inhibitory Potential of MBT-0206 and paclitaxel against the drug-sensitive human uterus sarcoma line Mes-SA.
**Figure 6 - 7****:** The growth inhibitory assay was performed in 24-well plates with each drug concentration tested in duplicate (n=2 wells). 4 x 10⁴ cells per well were seeded into a 24-well plate and incubated over night. The following day, 10-11 concentrations of the respective drug formulation were added for 72 h to cover the range depicted in the respective graphs. Finally, the cell viability was determined by a standard MTT-assay measuring the activity of mitochondrial dehydrogenases.

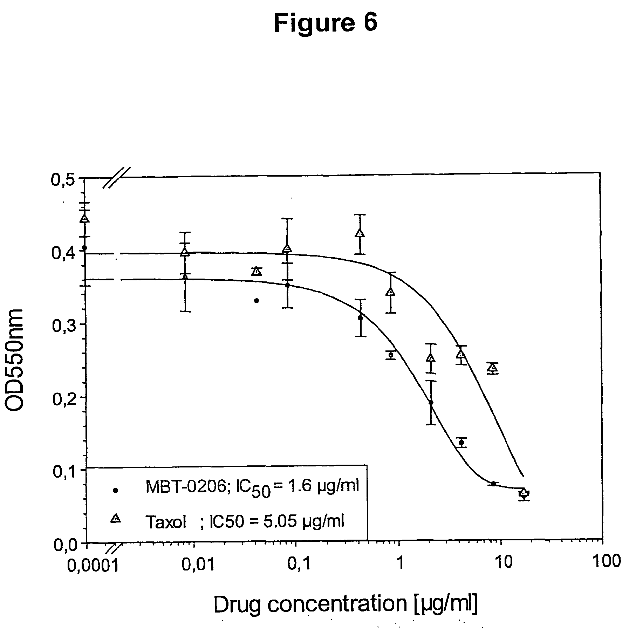

   **Figure 6****:** Inhibitory Potential of MBT-0206 and paclitaxel against the highly drug-resistant murine colon carcinoma line Colon-26_{MBT}.

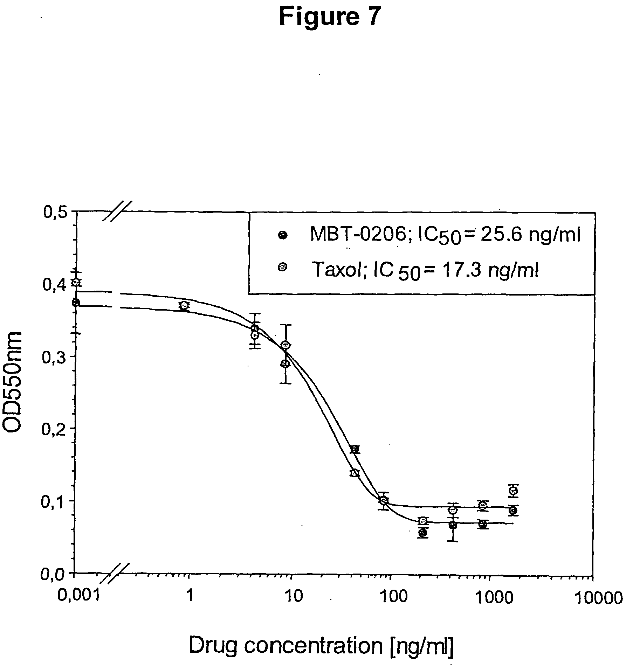

   **Figure 7****:** Inhibitory Potential of MBT-0206 and paclitaxel against the parental drug-sensitive murine colon carcinoma line Colon-26.

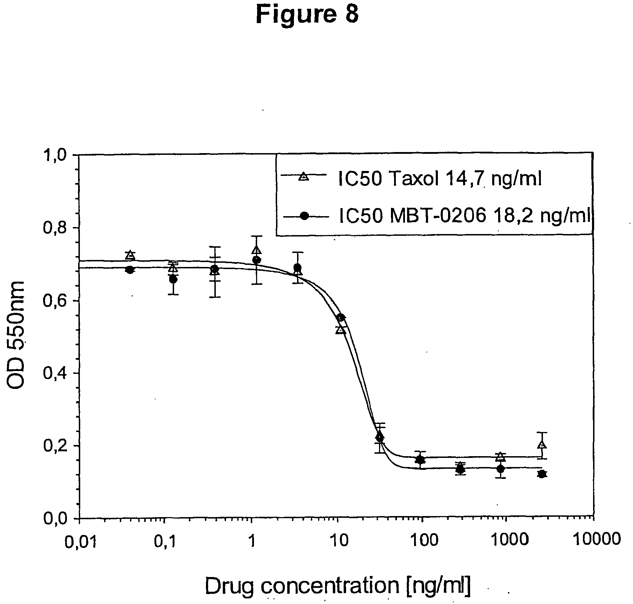

**Figure 8****:** Inhibitory Potential of MBT-0206 and paclitaxel against the drug-sensitive human endothelial line EA.hy926.
   The growth inhibitory assay was performed in 24-well plates with each drug concentration tested in duplicate (n=2 wells). 4 x 10⁴ cells per well were seeded into a 24-well plate and incubated over night. The following day, 11 concentrations of the respective drug formulation were added for 72 h to cover the range depicted in the respective graph. Finally, the cell viability was determined by a standard MTT-assay measuring the activity of mitochondrial dehydrogenases.

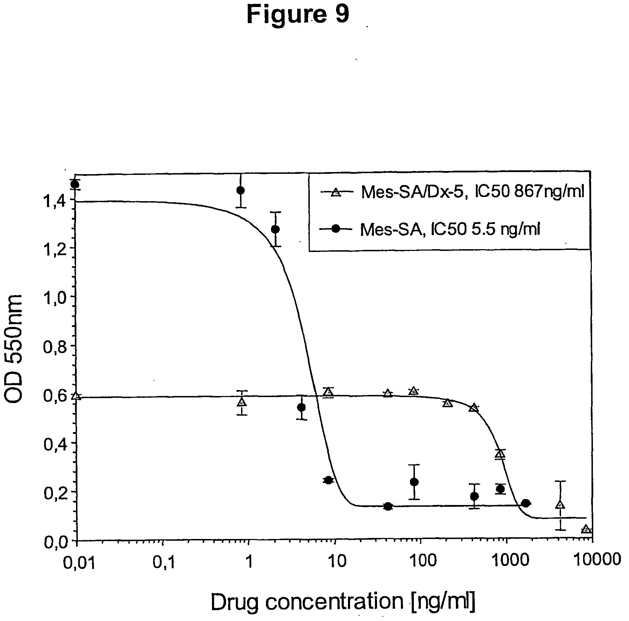

**Figure 9****:** In vitro inhibitory potential of paclitaxel against the sensitive parental line Mes-SA and the resistant derivative Mes-SA/Dx-5_{MBT} The growth inhibitory assay was performed in 24-well plates with each drug concentration tested in duplicate (n=2 wells). 4 x 10⁴ cells per well were seeded into a 24-well plate and incubated over night. The following day, 10-11 concentrations of the respective drug formulation were added for 72 h to cover the range depicted in the respective graphs. Finally, the cell viability was determined by a standard MTT-assay measuring the activity of mitochondrial dehydrogenases.

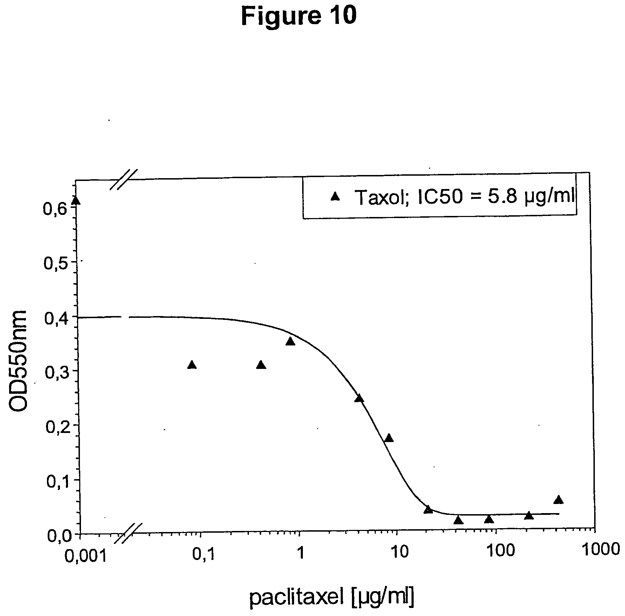

**Figure 10****:** Inhibitory Potential of paclitaxel against the drug-resistant human dermal melanoma line Sk-Mel28. The growth inhibitory assay was performed in 24-well plates with each drug concentration tested in duplicate (n=2 wells). 4 x 10⁴ cells per well were seeded into a 24-well plate and incubated over night. The following day, 11 concentrations of the respective drug formulation were added for 72 h to cover the range depicted in the respective graphs. Finally, the cell viability was determined by a standard MTT-assay measuring the activity of mitochondrial dehydrogenases.

The object of the invention is defined in the claims.

### Examples

### 1. Human Therapy Treatment Protocol

This example is concerned with human treatment protocols using the formulations disclosed. Treatment will be of use preventing and/or treating various human diseases and disorders associated with enhanced angiogenic activity. It is considered to be particularly useful in anti-tumor therapy, for example, in treating patients with solid tumors and hematological malignancies or in therapy against a variety of chronic inflammatory diseases such as rheumatoid arthritis or psoriasis.

A feature of the invention is that several classes of diseases and/or abnormalities may be treated by directly targeting angiogenic epithelial cells without directly targeting the tissue or cells. involved in the abnormality e.g., by inhibiting angiogenesis the blood supply to a tumor is cut off and the tumor is killed without directly targeting the tumor cells in any manner. Other classes of diseases and/or abnormalities may be treated by directly targeting angiogenic endothelial cells and by directly targeting the tissue or cells involved in the abnormality.

In an other application, drug resistant cells such as drug resistant cancer cells or highly proliferative synoviocytes in rheumatoid arthritis can be affected directly.

The various elements of conducting a clinical trial, including patient treatment and monitoring, will be known to those skilled in the art in light of the present disclosure.

For regulatory approval purposes, it is contemplated that patients chosen for a study would have failed to respond to at least one course of conventional therapy and would have objectively measurable disease as determined by physical examination, laboratory techniques, or radiographic procedures. Such patients would also have no history of cardiac or renal disease and any chemotherapy should be stopped at least 2 weeks before entry into the study.

Prior to application, the formulation can be reconstituted in an aqueous solution in the event that the formulation was freeze dried. As outlined above, the required application volume is calculated from the patient's body weight and the dose schedule.

The disclosed formulations may be administered over a short infusion time. The infusion given at any dose level should be dependent upon the toxicity achieved after each. Thus, if Grade II toxicity was reached after any single infusion, or at a particular period of time for a steady rate infusion, further doses should be withheld or the steady rate infusion stopped unless toxicity improved. Increasing doses should be administered to groups of patients until approximately 60% of patients showed unacceptable Grade III or IV toxicity in any category. Doses that are 2/3 of this value would be defined as the safe dose.

Physical examination, tumor measurements and laboratory tests should, of course, be performed before treatment and at intervals of about 3-4 weeks later. Laboratory tests should include complete blood cell counts, serum creatinine, creatine kinase, electrolytes, urea, nitrogen, SGOT, bilirubin, albumin and total serum protein.

Clinical responses may be defined by acceptable measure or changes in laboratory values e.g. tumor markers. For example, a complete response may be defined by the disappearance of all measurable disease for at least a month, whereas a partial response may be defined by a 50% or greater reduction.

All of the compositions and methods disclosed and claimed herein can be made and executed without undue experimentation in light of the present disclosure. While the compositions and methods of this invention have been described in terms of preferred embodiments, it will be apparent to those skilled in the art that variations may be applied to the composition, methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by the FDA Office of Biologics standards.

The present invention includes a method of delivery of a pharmaceutically effective amount of the inventive formulation of an active agent to a target site such as an angiogenic vascular target site of a subject in need thereof. A "subject in need thereof" refers to a mammal, e. g. a human.

The route of administration preferably comprises peritoneal or parenteral administration.

For use with the present invention the "pharmacologically effective amount" of a compound administered to a subject in need thereof will vary depending on a wide range of factors. The amount of the compound will depend upon the size, age, sex, weight, and condition of the patient, as well as the potency of the substance being administered. Having indicated that there is considerable variability in terms of dosing, it is believed that those skilled in the art can, using the present disclosure, readily determine appropriate dosing by first administering extremely small amounts and incrementally increasing the dose until the desired results are obtained. Although the amount of the dose will vary greatly based on factors as described above, in general, the present invention makes it possible to administer substantially smaller amounts of any substance as compared with delivery systems which only target the pathologic tissue e. g., target the tumor cells themselves.

### 2. Mono-therapy protocols

| **Study No.** | **Indication** |
|---|---|
| CTLP01 | Prostate Cancer |
| CTLP05 | Gastro-Intestinal Cancer |
| CTLP07 | Melanoma |
| CTLP09 | Breast Cancer |
| CTLP10 | Colorectal Cancer |
| CTLP15 | Breast Cancer |
| CTLP16 | Pancreatic Cancer |

### Dosing

| **Study No.** | **Dosages** |
|---|---|
| CTLP01 | 0.0688 mg/kg, 0.275 mg/kg, 1.10 mg/kg, 1.65 mg/kg (mg liposomal paclitaxel / kg) |
| CTLP05 | 0.275 mg/kg, 0.55 mg/kg, 1.10 mg/kg, 1.65 mg/kg (mg liposomal paclitaxel / kg) |
| CTLP07 | 0.275 mg/kg, 0.55 mg/kg, 1.10 mg/kg (mg liposomal paclitaxel / kg) |
| CTLP09 | 0.55 mg/kg, 1.10 mg/kg (mg liposomal paclitaxel / kg) |
| CTLP10 | 0.55 mg/kg (mg liposomal paclitaxel / kg) |
| CTLP15 | 25 mg/kg (mg liposomal paclitaxel / kg) |
| CTLP16 | 0.55 - 1.1 mg/kg (mg liposomal paclitaxel / kg) |

### Standard formulation liposomal paclitaxel

50 mol% DOTAP : 47 mol% DOPC : 3 mol% paclitaxel (MBT-0206 = EndoTag 1)

### Treatment schedule ongoing completed studies

| **Study No.** | **Schedule** | **No. of applications** |
|---|---|---|
| CTLP01 | 3 times a week | N=3 |
| CTLP05 | 3 times a week with 3 weeks interval each | N=6 |
| CTLP09 | 3 times a week with 2 weeks interval each | N=9 |
| CTLP10 | 3 times a week with 2 weeks interval each | N=9 |

### Treatment schedule ongoing studies

| **Study No.** | **Schedule** | **No. of applications** |
|---|---|---|
| CTLP03 | 3 times a week | N=3 |
| CTLP04 | weekly | N=14 |
| CTLP06 | 4-5 times a week with 1 week interval each | N=14 |
| CTLP07 | 3 times a week with 2-3 weeks interval each | N=6-9 |
| CTLP15 | 3 times a week with 2 weeks interval each | N=24 |
| CTLP16 | 3 times a week with 3 weeks interval each | N=9 |

### Efficacy

Response will be evaluated according to the WHO or RECIST criteria.

### 3. Combination therapy protocols

| **Study No.** | **Indication** |
|---|---|
| CTLP04 | Lung Cancer |
| CTLP06 | Colorectal or Gastric Cancer |

### Dosing

| **Study No.** | **Dosages** |
|---|---|
| CTLP04 | liposomal paclitaxel, 1.5 mg/kg and Carboplatin (2mg/ml/min i.v. For 15 min once weekly) |
| CTLP06 | liposomal paclitaxel, 0.5 or 1.0 mg/kg alone, 0.5 or 1.0 mg/kg liposomal paclitaxel and 5-fluorouracil (2000 mg/m²). |

### Standard formulation liposomal paclitaxel

50 mol% DOTAP : 47 mol% DOPC : 3 mol% paclitaxel

### Treatment schedule for liposomal paclitaxel in ongoing studies

| **Study No.** | **Schedule** | **No. of applications** |
|---|---|---|
| CTLP04 | once weekly | N=14 |
| CTLP06 | daily with one week terval each | N=14 |

A further planned study comprises administration of liposomal paclitaxel, e.g. 0.5 or 1.0 or 1.5 mg/kg and gemcitabine, e.g. 1000 mg/m² once weekly for three weeks followed by one week without treatment, preferably for an interval of at least one year.

The treatment schedule for liposomal paclitaxel will be as descibed above for ongoing studies.

### Efficacy

Response will be evaluated according to the WHO or RECIST criteria.

### 4. Case report #1

### Patient:

- 49 years old patient with large therapy resistant recidivism of a mucoepidermoidal carcinoma of the larynx
- metastases cervical, supraclavicular, axillar, mediastinal and pulmonal
- 5 years after first tumor resection, neck tumor dissection and adjuvant radiotherapy
- after repeated therapy of recidivism with multiple resections, plastic surgery, radiotherapy and chemotherapy

### Dosing schedule:

- MBT-0206: 50/47/3 (DOTAP/DOPC/paclitaxel)
- Application of 0.06, 0.25, 0.5 and 1.0 mg liposomal paclitaxel/kg bw, i.v.
- One cycle of 3 times a week (on day 1, 3 and 5)

### Results:

- Good tolerance while monitoring cardiovascular, pulmonary and serological parameter during and after infusion
- No signs of acute or chronic toxicity
- Reduction of tumor blood circulation
- Strongly reduced progression of tumor growth during 3 months

### 5. Case report #2

One patient with liver cell carcinoma, who had disease progression after multiple chemotherapies, has been treated with MBT-0206 .

Lyophilized MBT-0206 has been reconstituted with water for injection and a total infusion volume of 300-400 ml MBT-0206 (equivalent to a dose of 1.0 mg liposomal paclitaxel/kg body weight) has been administered by central or peripheral intravenous infusion over a period of 2-4h. The infusion rate has been increased slowly up to a maximum speed of 2,5 ml/min. Premedication depended on the patient's sex, age, condition. In the specific case of the above mentioned patient it has been given dexamethasone and an antihistamine.

MBT-0206 has been administered once weekly with a dose escalation schedule, beginning with 2 times 0.25 mg liposomal paclitaxel / kg bw, 1 times 0.5 mg liposomal paclitaxel/kg bw) and than a consolidation dose of 19 times 1.0 mg liposomal paclitaxel/kg bw. This treatment is after 22 weekly administrations still ongoing and up to now no adverse drug reactions have been reported. Besides the favourable safety profile the last evaluation of tumor size, which has been performed by CT-Scan of the liver, showed stable disease.

### 6. Case report #3

In another case a prostate cancer patient who became refractory to hormone therapy, has been treated with 1.0 mg liposomal paclitaxel/kg bw, 3 times weekly every third day under the same conditions of preparation and administration as described above. The premedication contained dexamethasone and antihistamines. The accumulated dose of liposomal paclitaxel for this patient in 7 days was 3.0 mg liposomal paclitaxel/kg bw.

### 7. Low dosing schedule with liposomal paclitaxel

Immortalised endothelial cells (EA.hy926) are seeded into 24-well plates (4 x 10⁴ cells per well) and grown over night. The following day, 9 wells are treated for 1 h with the low dose of 51.2 ng/ml liposomal paclitaxel (60 nM) formulated as MBT-0206. In addition, 3 wells per formulation are treated with the high dose of 153.7 ng/ml (180 nM) liposomal paclitaxel formulated as MBT-0206 for 1 h and 3 wells remain untreated. Approximately 24 h later, 6 of the 9 low dose-treated wells are again treated with the same low doses of MBT-0206 for 1 h (i.e. 2x treatment groups). Again 24 h later, 3 of these 6 two times -treated wells are treated for the third time with 51.2 ng/ml paclitaxel formulated as MBT-0206 for 1 h (3 x treatment groups). Approximately 96 h after this third treatment, the cell viability of all wells is quantitated. For this purpose, an assay which measures the activity of mitochondrial dehydrogenases using the tetrazolium salt 3-(4,5-dimethylthiazol-2-yl)-2,5-diphenyl tetrazolium bromide (MTT) is applied according to standard protocols (e.g. {Lindl, 1994 #28} with slight modifications).

The results demonstrate that the viability of cells treated for 3 times with a low dose of MBT-0206 is at least as strongly reduced as the viability of cells treated only one time with a high dose. Cells treated one time or two times with the low dose of MBT-0206 exhibit a somewhat increased viability which is, however, reduced in comparison to untreated cells.

### Conclusion

Treatments with high doses of MBT-0206 can be replaced by using low doses at a higher frequency. There is a correlation between treatment density (no. of treatments per week) and treatment efficacy. Three weekly treatments with low doses were superior to 1 or 2 weekly treatments. This optimised dosing regimen potentially reduces toxic side effects caused by high dose treatments.

### 8. Anti-tumor efficacy of MBT-0206 in combination with gemcitabine (50 mg/kg) in L3.6pl pancreatic tumors

### Animal model

Species: male Balb/c nu/nu mice
Tumor model: Solid orthotopic L3.6pl pancreas tumor (highly mestastatic; Bruns CJ, Harbison MT, Kuniyasu H, Eue I, Fidler IJ. In vivo selection and characterization of metastatic variants from human pancreatic adenocarcinoma by using orthotopic implantation in nude mice. Neoplasia 1999; 1(1):50-62.)
Supplier: Charles River France

### Treatment

Treatment start: Day 7 after tumor inoculation
Last treatment: Day 26 after tumor inoculation
Dose: MBT-0206 with 5 mg paclitaxel / kg bw per application paclitaxel at 5 mg paclitaxel / kg bw per application Gemcitabine (Gemzar) at 50 mg / kg per application
Schedule:
   MBT-0206, paclitaxel, trehalose: d 7, 10, 12, 14, 17, 19, 21, 24, 26
   Gemcitabine: d 7, 11, 14, 18, 21, 25
   Combination: combined mono treatments
Application route:
   MBT-0206, paclitaxel, trehalose: i. v. bolus into tail vein
   Gemcitabine: i. p. bolus

| **Groups (n = 9; n = 2-5 at day 26, 28)** | **Treatment** |
|---|---|
| Trehalose, paclitaxel, MBT-0206 | d 7, 10, 12, 14, 17, 19, 21, 24, 26 |
| Gemcitabine [50 mg/kg] | d 7, 11, 14, 18, 21, 25 |
| MBT-0206 [5 mg /kg bw] + | d 7, 10, 12, 14, 17, 19, 21, 24, 26 |
| Gemcitabine [50 mg/kg] | d 7, 11, 14, 18, 21, 25 |

The mortality was low before day 21: only 1 control and 1 MBT-0206 animal died. At day 24 the mortality increased for unknown reason: 3 MBT-0206, 1 Gemzar and 4 control animals were dead at day 24.

The body weight was not effected by either treatment, only the weight of control tumors decreased by 18 % during the last week.

### Monitored parameters

- Tumor volume palpated at day 10, 12, 14, 17, 19, 21, 24 after inoculation and at day 26, 28 after harvesting
- Necrospy after harvest at day 26 and 28

### Results

No effect of any treatment could be observed by palpation three days after begin of treatment. Strong anti-tumor effect was observed after one week and at day 24 by palpation, with the following ranking in efficacy: Gemzar-50 ≈ paclitaxel < MBT-0206 < MBT-0206 + Gemzar. However, after harvest at day 26, the measured tumor volumes of all groups were clearly lower compared to day 24. This difference in size is most likely due to imprecise palpation before harvest. At day 24 tumor size is reduced to ~ 30% by the mono treatments compared to the control group (n =2). The combination of MBT-0206 + Gemzar resulted in the strongest reduction of the tumor size to 13 %, which was significantly (p < 0.05) more effective compared to paclitaxel, Gemzar and MBT-0206 alone. At day 28 the control group (n =2) is not shown because one of the two tumors was extremely small compared all other tumors (day24, 26, 28) and thus considered as not representative. The tumors after the mono treatments showed a weak increase in tumor size compared to day 26 (paclitaxel: 536 mm³; MBT-0206: 392 mm³; Gemzar: 398 mm³), whereas the combination therapy led to a slight tumor regression between day 26 and 28 to 88 mm³.

These data show a strong anti-tumor efficacy of paclitaxel, Gemzar and MBT-0206 in this model. The anti-tumor action of MBT-0206 is slightly stronger than paclitaxel but similar to Gemzar. The combination of MBT-0206 and Gemzar shows an impressive anti-tumor efficacy.

### 9. Anti-tumor efficacy of MBT-0206 in combination with gemcitabine (100 mg/kg) in L3.6pl pancreatic tumors

### Animal model

Species: male Bald/c nu/nu mice
Tumor model: Solid orthotopic L3.6pl pancreas tumor (highly mestastatic) Supplier: Charles River France

### Treatment

Treatment start: Day 8 after tumor inoculation (01.05.03)
Last treatment: Day 26 after tumor inoculation (19.05.03)
Schedule:
MBT-0206, paclitaxel, trehalose: d 9, 12, 14, 16, 19, 21, 23, 26
Gemcitabine: d 8, 12, 15, 19, 22, 26
Combination: combined mono treatments

| **Groups (n = 9)** | **Treatment** |
|---|---|
| Trehalose, paclitaxel, MBT-0206 | d 9, 12, 14, 16, 19, 21, 23, 26 |
| Gemcitabine [100 mg/kg] | d 8, 12, 15, 19, 22, 26 |
| MBT-0206 [5 mg /kg bw] + | d 9, 12, 14, 16, 19, 21, 23, 26 |
| Gemcitabine [100 mg/kg] | d 8, 12, 15, 19, 22, 26 |

### Monitored parameters

- Tumor volume palpated at day 23, 26 after inoculation and after harvesting
- Body weight from day 1, 7 ,12, 16, 19, 21, 23, 27
- Necrospy after harvest at day 27

### Results (see figures 1 and 2)

Clear anti-tumor effect of all therapeutic treatments was observed at day 23 after tumor inoculation, with an prominent efficacy of the combination therapy (fig. 1). Ranking of tumor inhibition: paclitaxel < MBT-0206 = Gemzar-50 < MBT-0206 + Gemzar. Compared to the control group, the final tumor volumes were significantly reduced by MBT-0206 to 46% (p<0.05), by Gemzar to 47% (p<0.01) and by the combination therapy to 22% (p<0.01) at day 27. paclitaxel treatment reduced the final tumor volume to 68%, which was not significant. Interestingly, the efficacy of MBT-0206 and the combination therapy were more pronounced at day 23. Responsible for that might be the extended therapeutic interval during the weekend between day 23 and 27. These data reveal a clear anti-tumor efficacy of paclitaxel, Gemzar and MBT-0206 in this model. The anti-tumor action of MBT-0206 is slightly stronger than paclitaxel but similar to Gemzar Both MBT-0206 and the combination with Gemzar inhibited the formation of metastases (fig. 2). Liver metastases were absent only in the these two groups. Additionally, only in the combination group the lymph node metastases were rare. The data revealed that the combination of MBT-0206 and Gemzar enhances the anti-tumor efficacy of either mono-therapy.

The mortality was slightly increased in the treatment groups, particularly during combinatory treatment (4 mice died). No control animal died before harvest.

The body weight of control mice decreased by 18% during the last 11 days. During this period a transient decrease was also observed for the treated mice leading to a weight loss of 2% for paclitaxel, 12% for MBT-0206, 19% for the combination and 22% for Gemzar.

### 10. Killing of paclitaxel resistant cells (e.g. tumor cell lines)

To demonstrate the potential of MBT-0206 to directly kill tumors expressing (multi) drug resistance, two highly paclitaxel resistant mammalian tumor cell lines were investigated in vitro. These cell lines were selected by stepwise increasing the concentration of paclitaxel in the culture medium. Both cell lines have developed a high resistance level which is reflected by concentrations for 50 % growth inhibition (IC50 value) for paclitaxel around 1 or 5 µM (867 or 5000 ng/ml). In both instances, MBT-0206 is clearly superior to paclitaxel in killing drug resistant tumor cells. In contrast, in drug-sensitive or low-resistant cell lines, MBT-0206 has a more or less identical killing potential to paclitaxel.

### MBT 0206 and the human uterus sarcoma derived cell line Mes-SA and its derivative lines

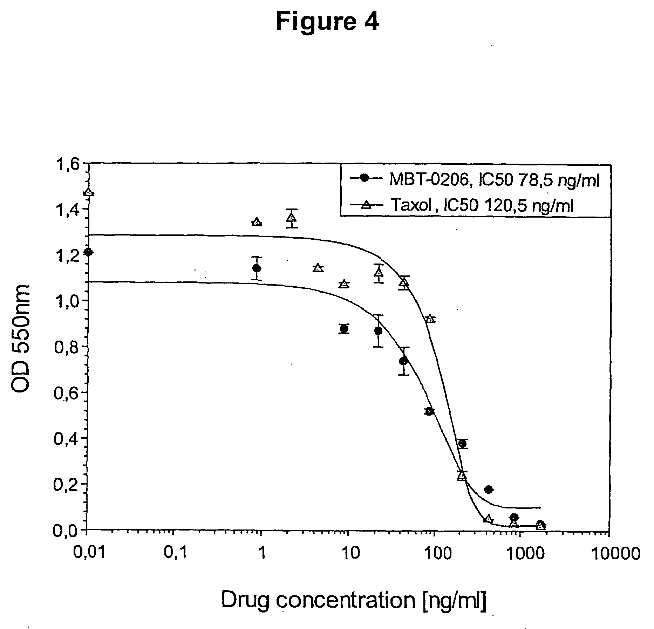

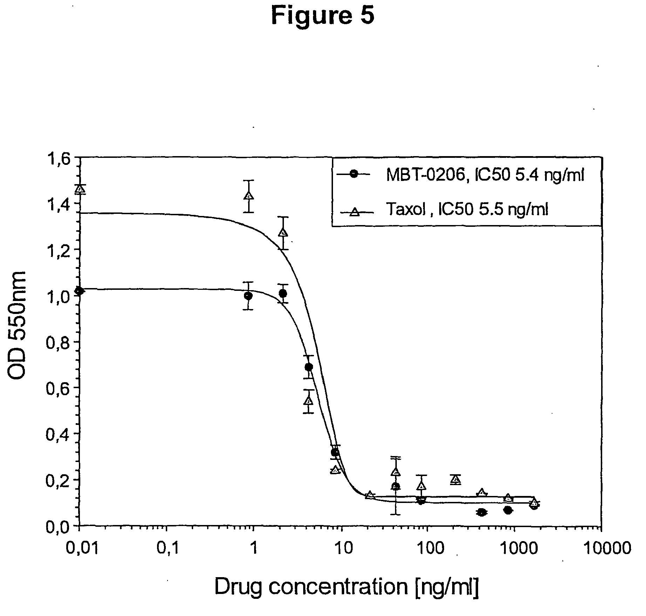

The highly paclitaxel resistant derivative cell line Mes-SA/Dx-5_{MBT} was selected with increasing paclitaxel concentrations from the commercially available line Mes-SA/Dx-5 (ATCC, {Harker, 1986 #29}). As shown in Fig. 3, it is highly resistant to paclitaxel indicated by the IC50 value of 867 ng/ml. Surprisingly, it is found that MBT-0206 is killing this cell line much more effectively, mirrored by the approximately 20-fold lower IC50 value. The commercially available line Mes-SA/Dx-5 which was selected from the parental line Mes-SA with doxorubicin ({Harker, 1986 #29}) expresses a low level of cross resistance for paclitaxel (compare Figs. 3 - 5). The IC50 value for paclitaxel is approximately 7-fold lower than in Mes-SA/Dx-5_{MBT}. Concomitantly, there is only a slight tendency of higher killing potential of MBT-0206 compared to paclitaxel in this cell line (Fig. 4). The parental line Mes-SA is highly sensitive for paclitaxel indicated by the low IC50 value of 5.5 ng/ml (Fig. 5). Against this drug-sensitive line, MBT-0206 has the same killing potential as paclitaxel. This is also true for all other paclitaxel-sensitive lines investigated so far. As example for this notion the results of treatments with MBT-0206 and paclitaxel of the immortalised endothelial line EA.hy926 are shown in Fig. 8.

### MBT-0206 and the murine colon carcinoma derived cell line Colon-26

In a similar way to Mes-SA/Dx-5_{MBT}, a highly paclitaxel resistant derivative line of the murine colon carcinoma line Colon-26 (Cell lines Service, Heidelberg) was established and called Colon-26_{MBT}. The IC50 value for paclitaxel is approximately 5 µg/ml (Fig. 6). Again as in Mes-SA/Dx-5_{MBT}, MBT-0206 had a clearly higher potential to inhibit the growth of this cell line. In this cell line, the IC50 values differ by a factor of 3. In line with the result shown for Mes-SA and EA.hy926 cells, the parental drug-sensitive line Colon-26 is equally sensitive for MBT-0206 and paclitaxel (Fig. 7).

### Conclusion

In highly paclitaxel-resistant cell lines, MBT-0206 has a significantly higher killing potential as paclitaxel. In paclitaxel-sensitive lines, both paclitaxel formulations have a comparable efficacy. MBT-0206 may therefore be able to kill also (multi) drug resistant tumors directly in vitro and in vivo. It may, therefore, be a new approach to treat human tumors (or other diseases) which become unresponsive for paclitaxel.

### 11. Therapy of recurrent head and neck squamous-cell carcinoma

A safety evaluation of paclitaxel loaded cationic liposomes (MBT-0206) in patients with recurrent, therapy refractory head and neck squamous-cell carcinoma was carried out.

### Study design

- Patients with recurrent, therapy refractory head and neck squamous-cell carcinoma
- Measurable disease, defined as at least one lesion that can be accurately measured in at least on dimension with spiral CT / MRI scan

- Karnofsky-performance index > 60 %
- Life expectancy > 4 months

### Drug Administration

- paclitaxel encapsulated in cationic liposomes was administered in 2 doses: 0.55 mg paclitaxel/kg and 1.10 mg paclitaxel/kg
- After a screening period, the drug was intravenously injected on day 1, 3 and 5

### Conclusions

### During and after injection no signs of acute or chronic toxicity were observed: Vital and lab safety parameters remained nearly constant.

The results indicate that the dose and schedule suggested from preclinical toxicology studies was well tolerated by the patients.

Cationic liposomes selectively targeted endothelium of human head and neck squamous cell carcinoma. Laser Doppler flowmetry confirmed the antivascular mechanism of action of the therapy.

### 12. MBT-0206 (EndoTag 1) is effective against paclitaxel-resistant tumors in vivo

Two tumor cell lines which show high survival rates upon 72 h treatments with paclitaxel in vitro were used for mouse tumor models to investigate the potential of MBT-0206 in vivo. In these tumor models, it can be demonstrated that MBT-0206 is inhibiting tumor growth significantly more effective than paclitaxel.
1. A highly paclitaxel resistant derivative (Mes-SA/Dx-5_{MBT}, IC50 0.87 µg/ml) was selected from the commercially available human uterus sarcoma derived cell Mes-SA/Dx-5 (ECACC). This moderately resistant line originates from the highly sensitive parental line Mes-SA. As shown in Fig. 9, the line Mes-SA/Dx-5_{MBT} exhibits a ~ 150-fold increased paclitaxel tolerance compared to the parental line Mes-SA as assessed by their respective IC50 values. For in vivo experiments, Mes-SA/Dx-5_{MBT} cells were injected subcutaneously (s.c.) into NMRI nude mice. Treatments with MBT-0206 or paclitaxel were started at day 12 and extended until day 21 with 3 weekly doses (5mg/kg b.w. paclitaxel). The mean tumor size of MBT-0206 treated animals was compared to the mean tumor size of paclitaxel treated animals 2 days after the last treatment (day 23 after start of treatments). The reduction induced by MBT-0206 compared to paclitaxel is given in % (see table below). As shown in the table below and Figure 9, MBT-0206 was clearly more effective than paclitaxel in this tumor model in reducing the tumor growth by ~ 1/3.
2. Concerning the human dermal melanoma line Sk-Mel28, already the commercially available line exhibits high paclitaxel resistance (IC50 5.8 µg/ml, Fig. 10) and no sensitive derivative line is available for comparison. In vivo, these cells were injected into SCID mice bearing human skin transplants between human skin transplant and mouse acceptor skin. Treatments were started on day 17 after tumor cell injection and extended to day 28 with doses (12.5 mg/kg b.w.) every second day. The therapeutic potential of MBT-0206 and paclitaxel were compared on day 7 after start of treatments (see table below). In this tumor model, MBT-0206 nearly completely blocked tumor development whereas paclitaxel was completely ineffective. Upon treatment with MBT-0206, tumors only marginally grew to about 1/10 the size of paclitaxel (paclitaxel)-treated tumors.

**Table**

| **Name of Tumor** | **Tumor reduction induced by MBT-0206 vs. paclitaxel (%)** | **No. of animals in group MBT-0206 or paclitaxel** |
|---|---|---|
| **Mes-SA/Dx-5_{MBT}** | 35.5 | 4/4 |
| **Sk-MeI28** | 90.0 | 6/5 |

Mes-SA/Dx-5_{MBT} tumor model: tumor volumes on day 23 after start of treatment (9 treatments, i.e. 3 per week, from day 12 to day 31 after tumor cell injection)

Sk-Mel tumor model: tumor volumes on day 7 after start of treatment (6 treatments very other day, i.e. from day 17 to day 28 after tumor cell injection).

The following items are part of the description:
1. Use of a cationic liposomal preparation comprising at least one cationic lipid from about 30 mole% to about 99.9 mole%, paclitaxel in an amount of at least about 0.1 mole% and at least one neutral and/or anionic lipid from about 0 mole % to about 70 mole% for manufacturing a pharmaceutical composition for administering to a human patient in need thereof at a monthly dose of about 0.25 mg up to about 60 mg of paclitaxel / kg body weight of said patient.
2. The use of claim 1, wherein said monthly dose is about 0.5 mg up to about 30 mg paclitaxel / kg body weight.
3. The use of claim 1 or 2, wherein said monthly dose is about 1.0 mg up to about 15 mg paclitaxel / kg body weight.
4. The use of claim 1 or 2, wherein said monthly dose is about 1 to about 7.5 mg/paclitaxel/kg body weight.
5. The use of claim 1 or 2, wherein said monthly dose is about 20 to about 60 mg/paclitaxel/kg body weight.
6. The use of any one of the claims 1 to 5, wherein administering said cationic liposomal preparation is at least once a time daily.
7. The use of any one of the claims 1 to 6, wherein administering said cationic liposomal preparation is a plurality of times during a month period, each of said times being separated by an interval of between one day and 3 weeks.
8. The use of any one of claims 1-7, wherein administering said cationic liposomal preparation is
   (i) at least 3 times, especially 3-5 times in a first week, followed by an interval of 1-3 weeks without administration, and optionally one or several repeats of this protocol,
   (ii) once in a first week followed by an interval of at least one week, especially 1-3 weeks, without administration, and optionally one or several repeats of this protocol,
   (iii) once in a week for one week or several successive weeks, or
   (iv) a combination of (i), (ii) and/or (iii).
9. Use of a cationic liposomal preparation comprising at least one cationic lipid from about 30 mole% to about 99.9 mole%, paclitaxel in an amount of at least about 0.1 mole% and at least one neutral and/or anionic lipid from about 0 mole % to about 70 mole% for manufacturing a pharmaceutical composition for simultaneous, separate, or sequential combination therapy with a jointly effective dose of at least one further active agent and/or heat and/or radiation and/or cryotherapy.
10. The use of claim 9, wherein the composition is for simultaneous combination therapy with a jointly effective dose of at least one further active agent.
11. The use of any one of the claims 1 to 10, wherein said cationic liposomal preparation comprises paclitaxel in an amount of at least about 2 mole% to about 8 mole%.
12. The use of any one of the claims 1 to 11, wherein said cationic liposomal preparation comprises paclitaxel in an amount of about 2.5 mole% to about 3.5 mole%.
13. The use of any one of the claims 1 to 17, wherein said cationic liposomal preparation comprises 50:47:3 mole% of DOTAP, DOPC and paclitaxel.
14. The use of any one of the claims 1 to 13, wherein said cationic liposomal preparation comprises substantially no paclitaxel crystals.
15. The use of any one of the claims 1 to 14 for treating an angiogenesis-associated condition.
16. The use of claim 15 for treating wound healing, cancer, an inflammatory disease or a chronic inflammatory disease such as rheumatoid arthritis, dermatitis, psoriasis or endometriosis.
17. Use of a cationic liposomal preparation comprising at least one cationic lipid from about 30 mole% to about 99.9 mole%, an active agent in an amount of at least about 0.1 mole% and at least one neutral and/or anionic lipid from about 0 mole % to about 70 mole% for manufacturing a pharmaceutical composition for the prevention or treatment of disorders associated with and/or accompanied by the occurrence of drug resistant cells, e.g. for the prevention or treatment of drug-resistant tumors.
18. The use of claim 17 as a second or third line treatment, particularly for cancer.
19. The use of claim 17 or 18, wherein said cationic liposomal preparation comprises 50:47:3 mole% of DOTAP, DOPC and paclitaxel.
20. Use of a cationic liposomal preparation comprising at least one cationic lipid from about 30 mole% to about 99.9 mole%, an active agent in an amount of at least about 0.1 mole% and at least one neutral and/or anionic lipid from about 0 mole % to about 70 mole% for manufacturing a pharmaceutical composition for the prevention or treatment of metastasis formation, e.g. onset and/or progression, particularly associated with and/or accompanied by a tumor disorder.
21. The use of claim 20 for manufacturing a pharmaceutical composition for the prevention or treatment of liver metastasis formation.
22. Use of a cationic liposomal preparation comprising at least one cationic lipid from about 30 mole% to about 99.9 mole%, an active agent in an amount of at least about 0.1 mole% and at least one neutral and/or anionic lipid from about 0 mole % to about 70 mole% for manufacturing a pharmaceutical composition for simultaneous, separate, or sequential combination therapy with a jointly effective dose of at least one further active agent and/or heat and/or radiation and/or cryotherapy against metastasis onset and/or progression, e.g. associated with and/or accompanied by the tumors.
23. The use of claim 22, wherein the composition is for simultaneous combination therapy with a jointly effective dose of at least one further active agent.
24. The use of any one of the claims 17 to 23, wherein said active agent is selected from a cytotoxic or cytostatic substance such as an anti-tumor or an anti-endothelial cell active substance, a chemotherapeutic agent or an immunological active substance.
25. The use of any one of claim 20-24, wherein said cationic liposomal preparation comprises 50:47:3 mole% of DOTAP, DOPC and paclitaxel.
26. The use of any one of claims 20-25, wherein said active agent is selected from a taxane, a camptothecin, a statin, a depsipeptide, thalidomide, other agents interacting with microtubuli such as discodermolide, laulimalide, isolaulimalide, eleutherobin, Sarcodictyin A and B, and in a most preferred embodiment it is selected from paclitaxel, docetaxel, camptothecin or any derivative thereof.
27. The use of claim 9 or 22, wherein said further active agent is an anti-endothelial cell active substance, an anti-tumor active substance, a chemotherapeutic agent, an immunological active substance, a compound that reduces or eliminates hypersensitivity reactions or a chemosensitizer.
28. The use of claims 9, 22 or 27, wherein said further active agent is selected from antineoplastic agents especially antimitotic agents like paclitaxel, alkylating agents especially platinum containing compounds like cisplatin, carboplatin, DNA topoisomerase inhibiting agents like camptothecin or doxorubicin, RNA / DNA antimetabolites, especially 5-fluorouracil or gemcitabine and other compounds having antitumor activity.
29. The use of claim 27, wherein said compound that reduces or eliminates hypersensitivity reactions is selected from the group comprising steroids, antihistamines, H2 receptor antagonists, and combinations thereof in a sufficient amount to prevent fatal anaphylactic reactions.
30. The use of claim 28, wherein said compound is selected from the group comprising Ranitidine, Dexamethasone, Diphenhydramine, Famotidine, Hydrocortisone, Clemastine, Cimetidine, Prednisolone, Chlorpheniramine, Chlorphenamine, Dimethindene maleate, and Promethazine.
31. The use of claim 27, wherein said chemosensitzier is selected from the group comprising cell cycle modulators, substances that revert a drug resistance like verapamil, vasoactive substances like anti-hypertensive drugs, substances that modify interactions of cationic liposomes with blood components like protamine.
32. The use of any one of claims 1-31 for the treatment of cancer, especially pancreatic cancer, inoperable pancreatic cancer, gastro-intestinal cancer, lung cancer, colorectal or gastric cancer, breast cancer, prostate cancer and melanoma.
33. The use of any one of the claims 1 to 32, wherein said cationic liposomal preparation comprises liposomes having an average particle diameter from about 25 nm to about 500 nm, preferably about 100 nm to about 300 nm.
34. The use of any one of the claims 1 to 30, wherein said cationic liposomal preparation is administered systemically, preferably intravenously.
35. Use of a cationic liposomal preparation comprising at least one cationic lipid from about 30 mole% to about 99.9 mole%, paclitaxel in an amount of at least about 0.1 mole% and at least one neutral and/or anionic lipid from about 0 mole % to about 70 mole% for manufacturing a pharmaceutical composition for administering to a human patient in need thereof at a monthly dose of about 9 mg up to about 2337 mg of paclitaxel/m² body surface of said human patient.

### Reference List

1. Rowinsky, E.K., and R.C. Donehower. 1995. paclitaxel (paclitaxel). N Engl J Med 332:1004-1014.
2. Awada, A. 2002. New cytotoxic agents and molecular-targeted therapies in the treatment of metastatic breast cancer. Forum (Genova) 12:4-15.
3. Seidman, A.D. 2003. Monotherapy options in the management of metastatic breast cancer. Semin Oncol 30:6-10.
4. Romanini, A., L. Tanganelli, F. Carnino, A. Fanucchi, R. Lionetto, S. Pastorino, S. Cosio, A. Gadducci, and P.F. Conte. 2003. First-line chemotherapy with epidoxorubicin, paclitaxel, and carboplatin for the treatment of advanced epithelial ovarian cancer patients. Gynecol Oncol 89:354-359.
5. Blom, R., N. Palm, and E. Simonsen. 1996. paclitaxel (paclitaxel) monotherapy in the treatment of progressive and recurrent ovarian carcinoma after platinum-based chemotherapy. Acta Oncol 35:733-736.
6. Modi, S., K.S. Panageas, E.T. Duck, A. Bach, N. Weinstock, J. Dougherty, L. Cramer, C. Hudis, L. Norton, and A. Seidman. 2002. Prospective exploratory analysis of the association between tumor response, quality of life, and expenditures among patients receiving paclitaxel monotherapy for refractory metastatic breast cancer. J Clin Oncol 20:3665-3673.
7. Ozols, R.F., B.N. Bundy, B.E. Greer, J.M. Fowler, D. Clarke-Pearson, R.A. Burger, R.S. Mannel, K. DeGeest, E.M. Hartenbach, and R. Baergen. 2003. Phase III trial of carboplatin and paclitaxel compared with cisplatin and paclitaxel in patients with optimally resected stage III ovarian cancer: a Gynecologic Oncology Group study. J Clin Oncol 21:3194-3200.
8. Vogelstein, B., E.R. Fearon, S.R. Hamilton, S.E. Kern, A.C. Preisinger, M. Leppert, Y. Nakamura, R. White, A.M. Smits, and J.L. Bos. 1988. Genetic alterations during colorectal-tumor development. N Engl J Med 319:525-532.
9. Kerbel, R.S. 1991. Inhibition of tumor angiogenesis as a strategy to circumvent acquired resistance to anti-cancer therapeutic agents. Bioessays 13:31-36.
10. Schünemann, Possinger, Scheidel, and Willich. 1999. Gynäkologische Malignome. Zuckschwerdt GmbH, Germering/München.
11. Heidemann, E., B. Steinke, and H.D. Waller. 1997. Therapieschemata Onkologie und Hämatologie. Urban & Schwarzenberg, München.
12. Heinemann, V. 2003. Role of gemcitabine in the treatment of advanced and metastatic breast cancer. Oncology 64:191-206.
13. Thigpen, J.T., J.A. Blessing, G. Olt, S.S. Lentz, and J. Bell. 2003. Cisplatin as second-line therapy in ovarian carcinoma treated initially with single-agent paclitaxel: a Gynecologic Oncology Group study. Gynecol Oncol 90:581-586.
14. Kuenen, B.C., L. Rosen, E.F. Smit, M.R. Parson, M. Levi, R. Ruijter, H. Huisman, M.A. Kedde, P. Noordhuis, W.J. van der Vijgh, G.J. Peters, G.F. Cropp, P. Scigalla, K. Hoekman, H.M. Pinedo, and G. Giaccone. 2002. Dose-finding and pharmacokinetic study of cisplatin, gemcitabine, and SU5416 in patients with solid tumors. J Clin Oncol 20:1657-1667.
15. Sledge, G.W., Jr. 2003. Gemcitabine combined with paclitaxel or paclitaxel/trastuzumab in metastatic breast cancer. Semin Oncol 30:19-21.
16. Reck, M., J. von Pawel, H.N. Macha, E. Kaukel, K.M. Deppermann, R. Bonnet, K. Ulm, S. Hessler, and U. Gatzemeier. 2003. Randomized phase III trial of paclitaxel, etoposide, and carboplatin versus carboplatin, etoposide, and vincristine in patients with small-cell lung cancer. J Natl Cancer Inst 95:1118-1127.
17. Zimpfer-Rechner, C., U. Hofmann, R. Figl, J.C. Becker, U. Trefzer, I. Keller, A. Hauschild, and D. Schadendorf. 2003. Randomized phase II study of weekly paclitaxel versus paclitaxel and carboplatin as second-line therapy in disseminated melanoma: a multicentre trial of the Dermatologic Co-operative Oncology Group (DeCOG). Melanoma Res 13:531-536.
18. Sledge, G.W., D. Neuberg, P. Bernardo, J.N. Ingle, S. Martino, E.K. Rowinsky, and W.C. Wood. 2003. Phase III trial of doxorubicin, paclitaxel, and the combination of doxorubicin and paclitaxel as front-line chemotherapy for metastatic breast cancer: an intergroup trial (E1193). J Clin Oncol 21:588-592.
19. Nobmann, S., B. Bauer, and G. Fricker. 2001. Ivermectin excretion by isolated functionally intact brain endothelial capillaries. Br J Pharmacol 132:722-728.
20. Thomas, H., and H.M. Coley. 2003. Overcoming multidrug resistance in cancer: an update on the clinical strategy of inhibiting p-glycoprotein. Cancer Control 10:159-165.
21. Harker, W.G., and B.I. Sikic. 1985. Multidrug (pleiotropic) resistance in doxorubicin-selected variants of the human sarcoma cell line MES-SA. Cancer Res 45:4091-4096.
22. Fellner, S., B. Bauer, D.S. Miller, M. Schaffrik, M. Fankhanel, T. Spruss, G. Bernhardt, C. Graeff, L. Farber, H. Gschaidmeier, A. Buschauer, and G. Fricker. 2002. Transport of paclitaxel (paclitaxel) across the blood-brain barrier in vitro and in vivo. J Clin Invest 110:1309-1318.
23. Kiesewetter, D.O., E.M. Jagoda, C.H. Kao, Y. Ma, L. Ravasi, K. Shimoji, L.P. Szajek, and W.C. Eckelman. 2003. Fluoro-, bromo-, and iodo paclitaxel derivatives: synthesis and biological evaluation. Nucl Med Biol 30:11-24.
24. Kohler, S., and W.D. Stein. 2003. Optimizing chemotherapy by measuring reversal of P-glycoprotein activity in plasma membrane vesicles. Biotechnol Bioeng 81:507-517.
25. Leonard, G.D., O. Polgar, and S.E. Bates. 2002. ABC transporters and inhibitors: new targets, new agents. Curr Opin Investig Drugs 3:1652-1659.
26. Agrawal, M., J. Abraham, F.M. Balis, M. Edgerly, W.D. Stein, S. Bates, T. Fojo, and C.C. Chen. 2003. Increased 99mTc-sestamibi accumulation in normal liver and drug-resistant tumors after the administration of the glycoprotein inhibitor, XR9576. Clin Cancer Res 9:650-656.
27. Callies, S., D.P. de Alwis, A. Harris, P. Vasey, J.H. Beijnen, J.H. Schellens, M. Burgess, and L. Aarons. 2003. A population pharmacokinetic model for paclitaxel in the presence of a novel P-gp modulator, Zosuquidar Trihydrochloride (LY335979). Br J Clin Pharmacol 56:46-56.
28. Lindl, T., and J. Bauer. 1994. Zell- und Gewebekultur. Gustav Fischer Verlag, Stuttgart.
29. Harker, W.G., D. Bauer, B.B. Etiz, R.A. Newman, and B.I. Sikic. 1986. Verapamil-mediated sensitization of doxorubicin-selected pleiotropic resistance in human sarcoma cells: selectivity for drugs which produce DNA scission. Cancer Res 46:2369-2373.

## Claims

1. A cationic liposomal preparation comprising at least one cationic lipid from about 30 mole% to about 99.9 mole %, paclitaxel in an amount of at least about 0.1 mole% and at least one neutral and/or anionic lipid from about 0 mole% to about 70 mole% for use in the administration to a human patient in need thereof in the treatment of an angiogenesis-associated condition selected from wound healing, cancer, an inflammatory disease, a chronic inflammatory disease selected from rheumatoid arthritis, dermatitis, psoriasis or endometriosis in an amount of about 0.01 to 2.5 mg of paclitaxel / kg body weight of said patient per single unit dose at a monthly dose of about 0.25 mg up to about 60 mg of paclitaxel / kg body weight.

2. The preparation for use of claim 1, wherein said monthly dose is about 1.0 mg up to about 15 mg paclitaxel / kg body weight.

3. The preparation for use of claim 1 or 2, wherein said monthly dose is about 1 to about 7.5 mg paclitaxel / kg body weight.

4. The preparation for use of any one of claims 1-3, wherein administering said cationic liposomal preparation is a plurality of times during a month period, each of said times being separated by an interval of between one day and 3 weeks.

5. The preparation for use of any one of claims 1-4, wherein administering said cationic liposomal preparation is
(i) at least 3 times, especially 3-5 times in a first week, followed by an interval of 1-3 weeks without administration, and optionally one or several repeats of this protocol,
(ii) once in a first week followed by an interval of at least one week, especially 1-3 weeks, without administration, and optionally one or several repeats of this protocol,
(iii) once in a week for one week or several successive weeks, or
(iv) a combination of (i), (ii) and/or (iii).

6. A cationic liposomal preparation comprising at least one cationic lipid from about 30 mole% to about 99.9 mole %, paclitaxel in an amount of at least about 0.1 mole% and at least one neutral and/or anionic lipid from about 0 mole% to about 70 mole% for use in simultaneous, separate, or sequential combination therapy of a human patient with a jointly effective dose of at least one further active agent, which is selected from antineoplastic agents, especially antimitotic agents selected from paclitaxel, alkylating agents, especially platinum containing compounds selected from cisplatin, carboplatin, DNA topoisomerase inhibiting agents selected from camptothecin or doxorubicin, RNA / DNA antimetabolites, especially 5-fluorouracil or gemcitabine and other compounds having antitumour activity, or a compound that reduces or eliminates hypersensitivity reactions comprising steroids, antihistamines, H2 receptor antagonists, and combinations thereof in a sufficient amount to prevent fatal anaphylactic reactions, or a compound comprising Ranitidine, Dexamethasone, Diphenhydramine, Famotidine, Hydrocortisone, Clemastine, Cimetidine, Prednisolone, Chlorpheniramine, Chlorphenamine, Dimethindene maleate, and Promethazine, or a chemosensitizer comprising cell cycle modulators, substances that revert a drug resistance selected from verapamil, vasoactive substances selected from anti-hypertensive drugs, substances that modify interactions of cationic liposomes with blood components selected from protamine, and/or heat and/or radiation and/or cryotherapy,
wherein the single unit dose of liposomal paclitaxel is in an amount of about 0.01 to 2.5 mg of paclitaxel / kg body weight of said patient.

7. The preparation for use of any one of claims 1 to 6, wherein said cationic liposomal preparation comprises paclitaxel in an amount of at least about 2 mole% to about 8 mole%.

8. The preparation for use of claims 1-7 for administration in wound healing, cancer, an inflammatory disease or a chronic inflammatory disease selected from rheumatoid arthritis, dermatitis, psoriasis or endometriosis.

9. A cationic liposomal preparation comprising at least one cationic lipid from about 30 mole% to about 99.9 mole%, an active agent in an amount of at least about 0.1 mole% and at least one neutral and/or anionic lipid from about 0 mole % to about 70 mole% for use in the prevention or treatment of disorders associated with and/or accompanied by the occurrence of drug resistant cells, e.g. for the prevention or treatment of drug-resistant tumors, of a human patient in an amount of about 0.01 to 2.5 mg of paclitaxel / kg body weight of said patient per single unit dose, wherein said active agent is paclitaxel.

10. A cationic liposomal preparation comprising at least one cationic lipid from about 30 mole% to about 99.9 mole%, an active agent in an amount of at least about 0.1 mole% and at least one neutral and/or anionic lipid from about 0 mole % to about 70 mole% for use in the prevention or treatment of metastasis formation, e.g. onset and/or progression, particularly associated with and/or accompanied by a tumour disorder, of a human patient in an amount of about 0.01 to 2.5 mg of paclitaxel / kg body weight of said patient per single unit dose, wherein said active agent is paclitaxel.

11. The preparation for use of any one of claim 1-10, wherein said cationic liposomal preparation comprises 50:47:3 mole% of DOTAP, DOPC and paclitaxel.

12. The preparation for use of any one of claims 1-11 for the treatment of cancer, especially pancreatic cancer, inoperable pancreatic cancer, gastro-intestinal cancer, lung cancer, colorectal or gastric cancer, breast cancer, prostate cancer and melanoma.

13. A cationic liposomal preparation comprising at least one cationic lipid from about 30 mole% to about 99.9 mole%, paclitaxel in an amount of at least about 0.1 mole% and at least one neutral and/or anionic lipid from about 0 mole % to about 70 mole% for use in the prevention or treatment of disorders associated with and/or accompanied by the occurrence of drug resistant cells, e.g. for the prevention or treatment of drug-resistant tumours, of a human patient.

14. A cationic liposomal preparation comprising at least one cationic lipid from about 30 mole% to about 99.9 mole%, paclitaxel in an amount of at least about 0.1 mole% and at least one neutral and/or anionic lipid from about 0 mole % to about 70 mole% for use in the prevention or treatment of metastasis formation, e.g. onset and/or progression, particularly associated with and/or accompanied by a tumour disorder, of a human patient.

## Patentansprüche

1. Kationische liposomale Zubereitung, umfassend mindestens ein kationisches Lipid von ungefähr 30 mol% bis ungefähr 99,9 mol%, Paclitaxel in einer Menge von mindestens ungefähr 0,1 mol% und mindestens ein neutrales und/oder anionisches Lipid von ungefähr 0 mol% bis ungefähr 70 mol% zur Verwendung für die Verabreichung an einen menschlichen Patienten, der dessen bedarf, bei der Behandlung eines Angiogenese-assoziierten Zustands, ausgewählt aus Wundheilung, Krebs, einer inflammatorischen Krankheit, einer chronisch inflammatorischen Krankheit ausgewählt aus rheumatoider Arthritis, Dermatitis, Psoriasis oder Endometriose in einer Menge von ungefähr 0,01 bis 2,5 mg Paclitaxel/kg Körpergewicht des Patienten pro einer Einheitsdosierung bei einer monatlichen Dosis von ungefähr 0,25 mg bis zu ungefähr 60 mg Paclitaxel/kg Körpergewicht.

2. Die Zubereitung zur Verwendung nach Anspruch 1, wobei die monatliche Dosis ungefähr 1,0 mg bis zu ungefähr 15 mg Paclitaxel/kg Körpergewicht ist.

3. Die Zubereitung zur Verwendung nach Anspruch 1 oder 2, wobei die monatliche Dosis ungefähr 1 bis ungefähr 7,5 mg Paclitaxel/kg Körpergewicht ist.

4. Die Zubereitung zur Verwendung nach einem der Ansprüche 1-3, wobei Verabreichen der kationischen liposomalen Zubereitung mehrmals während eines Zeitraums von einem Monat ist, wobei jedes dieser Male von einem Intervall zwischen einem Tag und 3 Wochen getrennt ist.

5. Die Zubereitung zur Verwendung nach einem der Ansprüche 1-4, wobei Verabreichen der kationischen liposomalen Zubereitung
(i) mindestens dreimal, insbesondere 3-5-mal in einer ersten Woche, gefolgt von einem Intervall von 1-3 Wochen ohne Verabreichung, und gegebenenfalls ein oder mehrere Wiederholungen dieses Protokolls,
(ii) einmal in einer ersten Woche, gefolgt von einem Intervall von mindestens einer Woche, insbesondere 1-3 Wochen, ohne Verabreichung und gegebenenfalls ein oder mehrere Wiederholungen dieses Protokolls,
(iii) einmal wöchentlich für eine Woche oder mehrere aufeinanderfolgende Wochen, oder
(iv) eine Kombination von (i), (ii) und/oder (iii),
ist.

6. Kationische liposomale Zubereitung, umfassend mindestens ein kationisches Lipid von ungefähr 30 mol% bis ungefähr 99,9 mol%, Paclitaxel in einer Menge von mindestens ungefähr 0,1 mol% und mindestens ein neutrales und/oder anionisches Lipid von ungefähr 0 mol% bis ungefähr 70 mol% zur Verwendung in gleichzeitiger, getrennter, oder sequenzieller Kombinationstherapie eines humanen Patienten mit einer gemeinschaftlich wirksamen Dosis mindestens eines zusätzlichen Wirkstoffs, der ausgewählt ist aus antineoplastischen Mitteln, insbesondere antimitotischen Mitteln, ausgewählt aus Paclitaxel, alkylierenden Mitteln, insbesondere Platin-enthaltende Verbindungen, ausgewählt aus Cisplatin, Carboplatin, DNA Topoisomerase-inhibierenden Mitteln, ausgewählt aus Camptothecin oder Doxorubicin, RNA/DNA-Antimetaboliten, insbesondere 5-Fluoruracil oder Gemcitabin und anderen Verbindungen mit Antitumor-Aktivität, oder einer Verbindung, die Hypersensitivitätsreaktionen reduziert oder eliminiert, umfassend Steroide, Antihistaminine, H2 Rezeptor-Antagonisten, und Kombinationen davon in einer ausreichenden Menge, um fatale anaphylaktische Reaktionen zu verhindern, oder eine Verbindung umfassend Ranitidin, Dexamethason, Diphenhydramin, Famotidin, Hydrocortison, Clemastin, Cimetidin, Prednisolon, Chlorpheniramin, Chlorphenamin, Dimethindenmaleat, und Promethazin, oder einen Chemosensibilisator, umfassend Zellzyklus-Modulatoren, Substanzen, die eine Arzneimittelresistenz umkehren, ausgewählt aus Verapamil, vasoaktiven Substanzen, ausgewählt aus anti-hypertensiven Arzneimitteln, Substanzen, die Interaktionen von kationischen Liposomen mit Blutbestandteilen modifizieren, ausgewählt aus Protamin, und/oder Wärme und/oder Bestrahlung und/oder Cryotherapie, wobei die eine Einheitsdosierung von liposomalem Paclitaxel in einer Menge von ungefähr 0,01 bis 2,5 mg Paclitaxel/kg Körpergewicht des Patienten ist.

7. Die Zubereitung zur Behandlung nach einem der Ansprüche 1 bis 6, wobei die kationische liposomale Zubereitung Paclitaxel in einer Menge von mindestens ungefähr 2 mol% bis ungefähr 8 mol% umfasst.

8. Die Zubereitung zur Verwendung nach Ansprüchen 1-7 zur Verabreichung bei Wundheilung, Krebs, einer inflammatorischen Krankheit oder einer chronisch inflammatorischen Krankheit ausgewählt aus rheumatoider Arthritis, Dermatitis, Psoriasis oder Endometriose.

9. Kationische liposomale Zubereitung, umfassend mindestens ein kationisches Lipid von ungefähr 30 mol% bis ungefähr 99,9 mol%, einen Wirkstoff in einer Menge von mindestens ungefähr 0,1 mol% und mindestens ein neutrales und/oder anionisches Lipid von ungefähr 0 mol% bis ungefähr 70 mol% zur Verwendung bei der Prävention oder Behandlung von Störungen, die mit dem Auftreten von Arzneimittel-resistenten Zellen assoziiert sind und/oder begleitet werden, z.B. zur Prävention oder Behandlung von Arzneimittel-resistenten Tumoren, eines humanen Patienten in einer Menge von ungefähr 0,01 bis 2,5 mg Paclitaxel/kg Körpergewicht des Patienten pro einer Einheitsdosierung, wobei der Wirkstoff Paclitaxel ist.

10. Kationische liposomal Zubereitung, umfassend mindestens ein kationisches Lipid von ungefähr 30 mol% bis ungefähr 99,9 mol%, einen Wirkstoff in einer Menge von mindestens ungefähr 0,1 mol% und mindestens ein neutrales und/oder anionisches Lipid von ungefähr 0 mol% bis ungefähr 70 mol% zur Verwendung bei der Prävention oder Behandlung von Metastasenbildung, z.B. Beginn und/oder Progression, insbesondere assoziiert mit und/oder begleitet von einer Tumor-Störung, eines humanen Patienten in einer Menge von ungefähr 0,01 bis 2,5 mg Paclitaxel/kg Körpergewicht des Patienten pro eine Einheitsdosierung, wobei der Wirkstoff Paclitaxel ist.

11. Die Zubereitung zur Verwendung nach einem der Ansprüche 1-10, wobei die kationische liposomale Zubereitung 50:47:3 mol% DOTAP, DOPC und Paclitaxel umfasst.

12. Die Zubereitung zur Verwendung nach einem der Ansprüche 1-11 zur Behandlung von Krebs, insbesondere Pankreaskrebs, inoperablem Pankreaskrebs, gastrointestinalem Krebs, Lungenkrebs, Dickdarm- oder Magenkrebs, Brustkrebs, Prostatakrebs und Melanom.

13. Kationische liposomale Zubereitung, umfassend mindestens ein kationisches Lipid von ungefähr 30 mol% bis ungefähr 99,9 mol%, Paclitaxel in einer Menge von mindestens ungefähr 0,1 mol% und mindestens ein neutrales und/oder anionisches Lipid von ungefähr 0 mol% bis ungefähr 70 mol% zur Verwendung bei der Prävention oder Behandlung von Störungen, die assoziiert sind mit und/oder begleitet werden durch das Auftreten Arzneimittel-resistenter Zellen, z.B. zur Prävention oder Behandlung von Arzneimittel-resistenten Tumoren, eines humanen Patienten.

14. Kationische liposomale Zubereitung, umfassend mindestens ein kationisches Lipid von ungefähr 30 mol% bis ungefähr 99,9 mol%, Paclitaxel in einer Menge von mindestens ungefähr 0,1 mol% und mindestens ein neutrales und/oder anionisches Lipid von ungefähr 0 mol% bis ungefähr 70 mol% zur Verwendung bei der Prävention oder Behandlung von Metastasenbildung, z.B. Beginn und/oder Progression, insbesondere assoziiert mit und/oder begleitet von einer Tumor-Störung, eines humanen Patienten.

## Revendications

1. Préparation liposomique cationique comprenant au moins un lipide cationique d'environ 30 % en mole à environ 99,9 % en mole, du paclitaxel dans une quantité d'au moins environ 0,1 % en mole et au moins un lipide neutre et/ou anionique d'environ 0 % en mole à environ 70 % en mole, pour utilisation dans l'administration à un patient humain en ayant besoin, dans le traitement d'une affection associée à une angiogenèse sélectionnée parmi la cicatrisation d'une blessure, un cancer, une maladie inflammatoire, une maladie inflammatoire chronique, sélectionnée parmi la polyarthrite rhumatoïde, la dermatite, le psoriasis ou l'endométriose, dans une quantité d'environ 0,01 à 2,5 mg de paclitaxel/kg de masse corporelle dudit patient, par dose unitaire unique, à une dose mensuelle d'environ 0,25 mg à environ 60 mg de paclitaxel/kg de masse de corporelle.

2. Préparation pour utilisation selon la revendication 1, dans laquelle ladite dose mensuelle est d'environ 1,0 mg à environ 15 mg de paclitaxel/kg de masse corporelle.

3. Préparation pour utilisation selon la revendication 1 ou 2, dans laquelle ladite dose mensuelle est d'environ 1 à environ 7,5 mg de paclitaxel/kg de masse corporelle.

4. Préparation pour utilisation selon l'une quelconque des revendications 1 à 3, dans laquelle l'administration de ladite préparation liposomique cationique se produit plusieurs fois pendant une période d'un mois, chacune desdites fois étant séparée par un intervalle d'entre un jour et 3 semaines.

5. Préparation pour utilisation selon l'une quelconque des revendications 1 à 4, dans laquelle l'administration de ladite préparation liposomique cationique s'effectue
(i) au moins 3 fois, en particulier 3 à 5 fois pendant une première semaine, suivie d'un intervalle de 1 à 3 semaines sans administration, et éventuellement une ou plusieurs répétitions de ce protocole,
(ii) une fois pendant une première semaine, suivie d'un intervalle d'au moins une semaine, en particulier d'1 à 3 semaines, sans administration, et éventuellement une ou plusieurs répétitions de ce protocole,
(iii) une fois par semaine pendant une semaine ou plusieurs semaines successives, ou
(iv) selon une combinaison de (i), (ii) et/ou (iii).

6. Préparation liposomique cationique, comprenant au moins un lipide cationique d'environ 30 % en mole à environ 99,9 % en mole, du paclitaxel dans une quantité d'au moins environ 0,1 % en mole et au moins un lipide neutre et/ou anionique d'environ 0 % en mole à environ 70 % en mole, pour utilisation dans une thérapie de combinaison simultanée, séparée ou séquentielle d'un patient humain, avec une dose conjointement efficace d'au moins un autre agent actif, qui est choisi parmi des agents antinéoplasiques, en particulier des agents antimitotiques choisis parmi le paclitaxel, des agents alkylants, en particulier des composés contenant du platine sélectionnés parmi le cisplatine, le carboplatine, des agents inhibiteurs de topoisomérase d'ADN sélectionnés parmi la camptothécine ou la doxorubicine, des antimétabolites d'ARN/ADN, en particulier du 5-fluorouracile ou de la gemcitabine et d'autres composés ayant une activité anti-tumorale, ou un composé qui réduit ou élimine les réactions d'hypersensibilité comprenant les stéroïdes, les antihistaminiques, les antagonistes de récepteur H2, et des combinaisons correspondantes dans une quantité suffisante pour prévenir des réactions anaphylactiques fatales ou un composé comprenant la Ranitidine, la Dexaméthasone, la Diphénhydramine, la Famotidine, l'Hydrocortisone, la Clémastine, la Cimétidine, la Prednisolone, la Chlorphéniramine, la Chlorphénamine, le maléate de dimétindène, et la Prométhazine, ou un chimio-sensibilisant comprenant des modulateurs du cycle cellulaire, des substances qui inversent une résistance au médicament sélectionnées parmi le vérapamil, des substances vasoactives sélectionnées parmi des médicaments antihypertenseurs, des substances qui modifient les interactions des liposomes cationiques avec les composants sanguins sélectionnées parmi la protamine, et/ou la chaleur et/ou la radiation et/ou la cryothérapie,
dans laquelle la dose unitaire unique de paclitaxel liposomique est dans une quantité d'environ 0,01 à 2,5 mg de paclitaxel/kg de masse corporelle dudit patient.

7. Préparation pour utilisation selon l'une quelconque des revendications 1 à 6, dans laquelle ladite préparation liposomique cationique comprend du paclitaxel dans une quantité d'au moins environ 2 % en mole à environ 8 % en mole.

8. Préparation pour utilisation selon l'une quelconque des revendications 1 à 7, pour administration dans la cicatrisation d'une blessure, un cancer, une maladie inflammatoire ou une maladie inflammatoire chronique sélectionnée parmi la polyarthrite rhumatoïde, la dermatite, le psoriasis ou l'endométriose.

9. Préparation liposomique cationique, comprenant au moins un lipide cationique d'environ 30 % en mole à environ 99,9 % en mole, un agent actif dans une quantité d'au moins environ 0,1 % en mole et au moins un lipide neutre et/ou anionique d'environ 0 % en mole à environ 70 % en mole, pour utilisation dans la prévention ou le traitement de troubles associés à et/ou accompagnés par la survenue de cellules résistantes aux médicaments, par exemple pour la prévention ou le traitement de tumeurs résistantes aux médicaments, d'un patient humain, dans une quantité d'environ 0,01 à 2,5 mg de paclitaxel/kg de masse corporelle dudit patient, par dose unitaire unique, dans laquelle ledit agent actif est le paclitaxel.

10. Préparation liposomique cationique comprenant au moins un lipide cationique d'environ 30 % en mole à environ 99,9 % en mole, un agent actif dans une quantité d'au moins environ 0,1 % en mole et au moins un lipide neutre et/ou anionique d'environ 0 % en mole à environ 70 % en mole, pour utilisation dans la prévention ou le traitement de la formation de métastases, par exemple leur apparition et/ou leur progression, en particulier associée à et/ou accompagnée par un trouble tumoral, d'un patient humain, dans une quantité d'environ 0,01 à 2,5 mg de paclitaxel/kg de masse corporelle dudit patient par dose unitaire unique, dans laquelle ledit agent actif est le paclitaxel.

11. Préparation pour utilisation selon l'une quelconque des revendications 1 à 10, dans laquelle ladite préparation liposomique cationique comprend 50:47:3 % en mole de DOTAP, DOPC et paclitaxel.

12. Préparation pour utilisation selon l'une quelconque des revendications 1 à 11, pour le traitement d'un cancer, en particulier du cancer du pancréas, du cancer du pancréas inopérable, du cancer gastro-intestinal, du cancer du poumon, du cancer colorectal ou gastrique, du cancer du sein, du cancer de la prostate et du mélanome.

13. Préparation liposomique cationique comprenant au moins un lipide cationique d'environ 30 % en mole à environ 99,9 % en mole, du paclitaxel dans une quantité d'au moins environ 0,1 % en mole et au moins un lipide neutre et/ou anionique d'environ 0 % en mole à environ 70 % en mole, pour utilisation dans la prévention ou le traitement de troubles associés à et/ou accompagnés par la survenue de cellules résistantes aux médicaments, par exemple pour la prévention ou le traitement de tumeurs résistantes aux médicaments, d'un patient humain.

14. Préparation liposomique cationique comprenant au moins un lipide cationique, d'environ 30 % en mole à environ 99,9 % en mole, du paclitaxel dans une quantité d'au moins environ 0,1 % en mole et au moins un lipide neutre et/ou anionique d'environ 0 % en mole à environ 70 % en mole, pour utilisation dans la prévention ou le traitement de la formation de métastases, par exemple leur apparition et/ou leur progression, en particulier associée à et/ou accompagnée par un trouble tumoral, d'un patient humain.
